(19) Europäisches Patentamt / European Patent Office / Office européen des brevets

(11) **EP 2 769 219 B1**

(12) **EUROPEAN PATENT SPECIFICATION**

(45) Date of publication and mention of the grant of the patent:
**30.08.2017 Bulletin 2017/35**

(21) Application number: **12780383.1**

(22) Date of filing: **22.10.2012**

(51) Int Cl.:
***G01N 33/543*** *(2006.01)*

(86) International application number:
**PCT/US2012/061354**

(87) International publication number:
**WO 2013/059805 (25.04.2013 Gazette 2013/17)**

(54) **POINT-OF CARE IMMUNOASSAY FOR QUANTITATIVE SMALL ANALYTE DETECTION**

POINT-OF-CARE IMMUNOVERFAHREN ZUM QUANTITATIVEN NACHWEISS VON KLEINEN ANALYTEN

TESTS DE POINT DE SOINS DE DETECTION IMMUNOLOGIQUE POUR MESURER QUANTITATIVEMENT DE PETITS ANALYTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2011 US 201161550141 P**
**23.04.2012 US 201261637143 P**

(43) Date of publication of application:
**27.08.2014 Bulletin 2014/35**

(73) Proprietor: **Decimadx LLC**
**Atlanta, GA 30363 (US)**

(72) Inventor: **GIBBS, Phillip**
**Atlanta, GA 30327 (US)**

(74) Representative: **Potter Clarkson LLP**
**The Belgrave Centre**
**Talbot Street**
**Nottingham NG1 5GG (GB)**

(56) References cited:
**WO-A2-2005/098439**
**WO-A2-2006/073500**
**WO-A2-2008/070865**

- **SUMEDHA D JAYASENA: "Aptamers: an emerging class of molecules that rival antibodies in diagnostics", CLINICAL CHEMISTRY, AMERICAN ASSOCIATION FOR CLINICAL CHEMISTRY, WASHINGTON, DC, vol. 45, 1 January 1999 (1999-01-01), pages 1628-1650, XP008156390, ISSN: 0009-9147**
- **Eun Jeong Cho ET AL: "Applications of Aptamers as Sensors", Annual Review of Analytical Chemistry, vol. 2, no. 1, 19 July 2009 (2009-07-19), pages 241-264, XP55036844, ISSN: 1936-1327, DOI: 10.1146/annurev.anchem.1.031207.112851**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The invention is generally related to an assay method for detecting the presence of an analyte in a sample and devices and kits for performing same.

### BACKGROUND OF THE INVENTION

Prescription opioid abuse and addiction are taking a rapidly growing toll on individuals, institutions and businesses in the United States. It has been estimated that nearly 2.5 million individuals initiate the nonmedical use of prescription opioids each year, and incidence of prescription opioid abuse now exceeds that of many conventional street drugs, including cocaine and heroin. Opioid prescriptions can be misused by a wide range of methods. Patients may seek prescription opioids for pain symptoms that are real, exaggerated, or nonexistent, visiting multiple physicians and filling the prescriptions at multiple pharmacies, a practice known as "doctor shopping." These prescriptions may then be misused by the patients themselves, diverted to family members or friends, or sold on the black market.

With prescription drug abuse on the rise, it is important for health care providers to have a point-of-care method of identifying patients that are misusing opioids and/or other prescription drugs before providing additional prescriptions. Drug tests are currently available for detecting opioids in urine, hair, saliva, or blood. However, these assays are either not suitable for point-of-care detection or are not sufficiently quantitative. The same issues apply to other types of illegal drugs such as tetrahydrocannibinol ("THC"), the active ingredient in marijuana.

There are other small analyte point-of-care needs outside of the drug abuse context. For example, an individual's ability to metabolize nicotine has been shown to negatively correlate with their ability to respond to nicotine treatment. Smokers with reduced nicotine metabolism have higher blood nicotine levels and compensate for this by smoking less. These individuals also demonstrate higher levels of cessation in transdermal nicotine therapy trials. Conversely, individuals with a normal metabolic rate tend to smoke more and have lower cessation rates. These normal metabolizers may be candidates for higher-dose nicotine replacement, which might potentially give rise to adverse effects in those with impaired nicotine metabolism. A need therefore exists for a point-of-care assay to measure nicotine metabolism in a subject.

Within the context of toxicity, heavy metals become toxic when they are not metabolized by the body and accumulate in the soft tissues. Heavy metal toxicity can result in damaged or reduced mental and central nervous function, lower energy levels, and damage to blood composition, lungs, kidneys, liver, and other vital organs. Long-term exposure may result in slowly progressing physical, muscular, and neurological degenerative processes that mimic Alzheimer's disease, Parkinson's disease, muscular dystrophy, and multiple sclerosis. Allergies are not uncommon and repeated long-term contact with some metals or their compounds may even cause cancer. For some heavy metals, toxic levels can be just above the background concentrations naturally found in nature. Therefore, testing is essential. Several analytical methods are available to analyze the level of heavy metals, such as lead, in biological samples. The most common methods employed are flame atomic absorption spectrometry (AAS), graphite furnace atomic absorption spectrometry (GFAAS), anode stripping voltametry (ASV), inductively coupled plasma-atomic emission spectroscopy (ICP/AES), and inductively coupled plasma mass spectrometry (ICP/MS). However, these laboratory methods are labor-intensive, time-consuming, and expensive.

There are other needs for point-of-care assays that are not currently available for non-human uses, especially in the veterinary areas. For example, pregnancy checking in livestock and domestic animals requires obtaining blood samples and shipping of the sample to a lab to measure hormone levels to assess pregnancy, or conducting an ultrasound exam, which requires specialized training and equipment. It would be much less expensive and efficient if point-of-care assays were available for measurements of biological samples at the site of collection.

Enzyme-mediated immunoassays are frequently used as an initial evaluation drug/hormone testing, especially using samples. Such assays can test for numerous drugs or drug classes, can determine if a class of substances is present or absent, and typically show adequate sensitivity. However, these assays are not specific and fail to distinguish between different drugs of the same class. Christo, et al., Pain Physician, 14:123-143 (2011).

It is an object of the invention to provide a point-of-care assay for quantitatively measuring the amount of small analyte, such as a drug of abuse, heavy metal, or hormone, in a biological sample from a subject at the place of collection to provide immediate results.

It is also an object of the present invention to provide kits for a point-of-care assay for measuring the amount of small analyte in a biological sample.

## SUMMARY OF THE INVENTION

A point-of-care assay has been developed for quantitatively measuring the amount of a small analyte in a biological sample from a subject. The analytes may be organic, inorganic, or organometallic compounds, or metal ions. Exemplary analytes include drugs, metabolites, biologics such as hormones, toxins, and environmental contaminants.

The assay can be either a competitive or non-competitive assay. However, in preferred embodiments, the assay is a non-competitive immunoassay, which typically involves the use of a binding agent and a capture agent that simultaneously bind the analyte in a sandwich assay. Low molecular weight analytes are not large enough for simultaneous binding using routine reagents such as sandwich assays which rely on two antibodies recognizing different epitopes of an antigen. In some embodiments, the non-competitive assay involves the use of a "binding agent" that selectively binds the analyte, forming a "capture complex" of the binding agent and the analyte, and a "capture agent" that selectively binds the capture complex but not free analyte, forming a "sandwich complex." In these embodiments, the amount of sandwich complex is directly related to the amount of analyte in the sample. The assay is capable of simultaneous detection of multiple analytes for multiplex analysis and quantitative control.

The assay generally involves combining the biological sample with an assay fluid, a drug binding agent that specifically binds a drug analyte, a calibration/control analyte, and a calibration/control binding agent that specifically binds the calibration analyte. Exemplary binding agents and capture agents include antibodies, nucleic acid aptamers, and peptide aptamers that specifically bind analyte or capture complex, respectively. The binding agents are preferably linked to detectable labels, e.g., fluorescent labels, to facilitate detection of the sandwich complex. The capture agent may also be directly or indirectly linked to a detectable label to normalize detection parameters, e.g., light intensity for fluorescent labels. In some preferred embodiments, only the mobile element contains a label.

In some embodiments, the binding agent or capture agent is a nucleic acid aptamer beacon linked to a fluorophore and quencher pair such that quenching or unquenching occurs when the capture complex or sandwich complex is formed. In a preferred embodiment, the binding agent is an aptamer and the capture agent is an antibody. In other preferred embodiments, the binding agent an antibody and the capture agent is an aptamer.

In some embodiments, fluorescent molecules on the binding agents and capture agents form a fluorescence resonance energy transfer (FRET) donor-acceptor pair. In FRET, energy from a molecular fluorophore (donor) is excited to a high-energy state and transferred to another fluorophore (acceptor) via intermolecular dipole-dipole coupling.

The assay is preferably a lateral flow immunoassay. Lateral flow immunoassays typically involve a membrane strip with an application point (e.g., a sample pad), an optional conjugate zone, a capture zone, and an absorption zone (e.g. wicking pad). A particularly preferred membrane strip is FUSION 5™ (Whatman Inc.), which can perform all the functions of a lateral flow strip on a single material. A biological sample, optionally combined with an assay fluid, is added to the application point at the proximal end of the strip, and the strip is maintained under conditions which allow the sample to transport by capillary action through the strip to and through the capture zone.

In some embodiments, the binding agents are added to the biological sample prior to administration to the application point of the membrane strip. In other embodiments, the sample migrates through the conjugate zone, where binding agents have been immobilized. The sample re-mobilizes the binding agents, and the analyte in the sample interacts with the binding agents to form capture complexes. The capture complexes then migrate into the capture zone where one or more capture agents have been immobilized. Excess reagents move past the capture lines and are entrapped in the wicking pad.

The capture agents are preferably coated onto, or linked (using for example, covalent linkage) to capture particles that are physically trapped within the membrane. The capture agents be conjugated directly to the membrane. The capture zone may be organized into one or more capture lines containing capture agents. In preferred embodiments, the capture zone contains a plurality of capture lines for multiplex analysis, i.e., detection of two or more analytes. In addition, the capture zone may contain one or more control capture lines for detecting the presence of control analyte. The control analyte can be a dilution control, i.e., an analyte such as creatine that is typically present in the biological sample at predictable concentrations. The control analyte may also be a reference analyte at a known concentration used to provide quantitative correlations between label detection and analyte amounts.

The assay may also involve the use of a sample collection apparatus that is not in fluid contact with the solid phase apparatus. The sample collection apparatus may contain the binding agents. In certain embodiments, the binding agents are evaporatively-dried, vacuum-dried or freeze-dried in the sample collection apparatus.

Quantitative measurements may be obtained by plotting results against a response surface calculated from a plurality of analyte standards and adjusted using internal controls. For example, to determine the amount of analyte in the sample, the amount of sandwich complex in each capture line of the capture zone is assessed by measuring the detectable labels linked to the binding or capture agents.

In some embodiments, detectable label immobilized in or on the membrane (e.g., coated on capture particles trapped within the membrane) may be used to normalize detection parameters, e.g., light intensity for fluorescent labels. In these embodiments, the ratio of detectable label on binding agents to that of those immobilized in or on the membrane

is preferably plotted against a response surface calculated from a plurality of analyte standards. In preferred embodiments, three or more internal standard analytes (needed to detect curvature) are detected concurrently with unknown analytes and used to adjust the predetermined response surface to minimize error for that particular assay run.

The disclosed lateral flow immunoassay provides a fast and accurate determination of the amount of a small analyte (e.g., drug, drug metabolite, heavy metal, or hormone) in a biological sample at the place of collection to provide immediate results.

## BRIEF DESCRIPTION OF THE DRAWINGS

Figures 1A to 1B are illustrations of a small analyte detection method using antibodies that specifically bind the analyte (binding agent), and DNA or RNA aptamers that specifically bind the antibody/analyte conjugate (metatype) (i.e., capture agent). In Figure 1A, the antibody is shown bound to a fluorescent marker with an emission wavelength of Em2, and the aptamer is shown conjugated to an immobilized fluorescent particle with an emission wavelength of Em1. In Figure 1B, the aptamer is shown conjugated to a fluorescent marker with an emission wavelength of Em2, and the antibody is shown conjugated to an immobilized fluorescent particle with an emission wavelength of Em1. The analyte is bound by the antibody, which is then captured by the aptamer. Excitation wavelengths Ex1 or Ex2 may then be used to detect fluorescent particle (as a control) and fluorescent marker, respectively.

Figure 2A is an illustration of a small analyte detection method using antibodies that specifically bind the analyte (binding agent) and protein aptamers that specifically bind the antibody/analyte conjugate (metatype) (i.e., capture agents). In Figure 2A, the antibody is shown bound to a fluorescent marker with an emission wavelength of Em2, and the aptamer is shown conjugated to an immobilized fluorescent particle with an emission wavelength of Em1. Figure 2B is an illustration of small analyte detection method using protein aptamers that specifically bind the analyte (binding agent) and antibodies that specifically bind the aptamer/analyte conjugate (metatype) (i.e., capture agents). In Figure 2B, the aptamer is shown bound to a fluorescent marker with an emission wavelength of Em2, and the antibody is shown conjugated to a fluorescent particle with an emission wavelength of Em1.

Figures 3A-3B are illustrations of a small analyte detection method using a DNA/RNA complex and an aptamer (Fig. 3A) or an antibody (Fig. 3B) that together bind analyte. The DNA/RNA complex is shown conjugated to a quenched fluorescent marker that is unquenched with an emission wavelength of Em2 when bound to analyte and aptamer (Fig. 3A) or antibody (Fig. 3B). The aptamer (Fig. 3A) and antibody (Fig. 3B) are shown conjugated to an immobilized fluorescent particle with an emission wavelength of Em1. Figure 3C is an illustration of a small analyte detection method using antibodies that specifically bind the analyte (binding agent)and a DNA/RNA folding aptamer beacon that specifically bind the antibody/analyte conjugate (i.e, capture agent). The DNA/RNA complex is shown conjugated to an immobilized fluorescent particle with an emission wavelength of Em1 and also conjugated to a quenched fluorescent marker that is unquenched with an emission wavelength of Em2 when bound to analyte and antibody.

Figure 4 is an illustration of a lateral flow device made from a membrane strip having an application point at the proximal end, followed by a conjugation zone, a capture zone, and an absorbent zone. The arrow shows the direction of lateral flow from the proximal to distal end. A plurality of capture lines are shown in the capture zone.

## DETAILED DESCRIPTION OF THE INVENTION

A point-of-care assay is disclosed that can be used to quantitatively measure one or more small analytes (e.g., drug, drug metabolite, heavy metal, or hormone) in a biological sample from a patient or a domestic animal or livestock at the place of collection. In particular, the point-of-care assay allows a physician to determine a subject’s drug, drug metabolite, heavy metal, and/or hormone levels prior to prescribing any medication. In preferred embodiments, this assay can be done within 1 hour, preferably within 30 minutes, more preferably within 10 minutes of obtaining the biological sample.

### I. Definitions

The term "assay" refers to an *in vitro* procedure for analyzing a sample to determine the presence, absence, or quantity of one or more analytes of interest.

The terms "control" and "calibration" as used in connection with analytes, are used interchangeably to refer to analytes used as internal standards.

The term "analyte" refers to a chemical substance of interest that is a potential constituent of a biological sample and is to be analyzed by an assay.

The term "small analyte" refers to an analyte that is too small to be specifically bound by two antibodies that

are specific for the analyte. For example, a small analyte may have a molecular weight of less than 2,000 Daltons, more preferably less than 1,500 Daltons, most preferably less than 1,000 Daltons. The small molecule can be a hydrophilic, hydrophobic, or amphiphilic compound.

The term "opioid" refers to a chemical that works by binding to opioid receptors. The term includes natural opiates as well as synthetic and semi-synthetic opioids.

The term "opioid metabolite" refers to a product of opioid metabolism in the patient.

The term "heavy metal" refers to a metal with a specific gravity that is at least five times the specific gravity of water.

A "lateral flow" assay is a device intended to detect the presence (or absence) of a target analyte in sample in which the test sample flows along a solid substrate via capillary action.

The term "membrane" as used herein refers to a solid substrate with sufficient porosity to allow movement of antibodies or aptamers bound to analyte by capillary action along its surface and through its interior.

The term "membrane strip" or "test strip" refers to a length and width of membrane sufficient to allow separation and detection of analyte.

The term "application point" is the position on the membrane where a fluid can be applied.

The term "binding agent" refers to a compound that specifically binds to an analyte. The term "capture agent" refers to an immobilized compound that selectively binds analyte complexed with binding agent (capture complex) or free binding agent (as a control). The capture agent may be conjugated to an immobilized capture particle. Binding agents and capture agents may be linked (directly or indirectly) to a detectable label. A binding agent is indirectly linked to a detectable label if it is bound to a particle that is directly linked to the detectable label. Binding agents and capture agents include antibodies, nucleic acid aptamers, and peptide aptamers.

The term "capture complex" refers to a complex formed by the specific binding of a binding agent to an analyte. The capture complex is immobilized for detection when captured by an immobilized capture agent.

The term "sandwich complex" refers to a complex formed by the specific binding of an immobilized capture agent to a binding agent and an analyte.

The term "immobilized" refers to chemical or physical fixation of an agent or particle to a location on or in a substrate, such as a membrane. For example, capture agents may be chemically conjugated to a membrane, and particles coated with capture agents may be physically trapped within a membrane.

The term "capture particle" refers to a particle coated with a plurality of capture agents. In preferred embodiments, the capture particle is immobilized in a defined capture zone.

The term "capture zone" refers to a point on a membrane strip at which one or more capture agents are immobilized.

The term "sandwich assay" refers to a type of immunoassay in which the analyte is bound between a binding agent and a capture agent. The capture agent is generally bound to a solid surface (e.g., a membrane or particle), and the binding agent is generally labeled.

The term "antibody" refers to intact immunoglobulin molecules, fragments or polymers of immunoglobulin molecules, single chain immunoglobulin molecules, human or humanized versions of immunoglobulin molecules, and recombinant immunoglobulin molecules, as long as they are chosen for their ability to bind an analyte.

The term "aptamer" refers to an oligonucleic acid or peptide molecule that binds to a specific target molecule. Aptamers are generally selected from a random sequence pool. The selected aptamers are capable of adapting unique tertiary structures and recognizing target molecules with high affinity and specificity.

A "nucleic acid aptamer" is an oligonucleic acid that binds to a target molecule via its conformation. A nucleic acid aptamer may be constituted by DNA, RNA, or a combination thereof. Nucleic acid aptamers are typically engineered using SELEX (systematic evolution of ligands by exponential enrichment).

A "peptide aptamer" is a combinatorial peptide molecule with a randomized amino acid sequence that is selected for its ability to bind a target molecule. Peptide aptamers are typically selected from combinatorial peptide libraries using yeast two-hybrid or phage display assays.

The term "metatype" refers to the analyte-binding site of a binding agent when bound to analyte. The term "idiotype" refers to the analyte binding site of a binding agent free of its analyte.

The term "anti-metatype" refers to a binding agent that selectively recognizes a binding agent-analyte complex (metatype) but lacks specificity for either free analyte or free binding agent. The term "anti-idiotype" refers to a binding agent that selectively recognizes the analyte binding site of another binding agent.

The term "specifically binds" or "selectively binds" refers to a binding reaction which is determinative of the presence of the analyte in a heterogeneous population. Generally, a first molecule that "specifically binds" a second molecule has an affinity constant ($K_a$) greater than about $10^5$ $M^{-1}$ (e.g., $10^6$ $M^{-1}$, $10^7$ $M^{-1}$, $10^8$ $M^{-1}$, $10^9$ $M^{-1}$, $10^{10}$ $M^{-1}$, $10^{11}$ $M^{-1}$, and $10^{12}$ $M^{-1}$ or more) with that second molecule.

The term "detectable label" refers to any moiety that can be selectively detected in a screening assay. Examples include radiolabels, (e.g., $^3H$, $^{14}C$, $^{35}S$, $^{125}I$, $^{131}I$), affinity tags (e.g. biotin / avidin or streptavidin), binding sites for

antibodies, metal binding domains, epitope tags, fluorescent or luminescent moieties (e.g. fluorescein and derivatives, green fluorescent protein (GFP), rhodamine and derivatives, lanthanides), colorimetric probe, and enzymatic moieties (e.g. horseradish peroxidase, β-galactosidase, β-lactamase, luciferase, alkaline phosphatase).

The term "biological sample" refers to a tissue (e.g., tissue biopsy), organ, cell, cell lysate, or body fluid from a subject. Non-limiting examples of body fluids include blood, urine, plasma, serum, tears, lymph, bile, cerebrospinal fluid, interstitial fluid, aqueous or vitreous humor, colostrum, sputum, amniotic fluid, saliva, anal and vaginal secretions, perspiration, semen, transudate, exudate, and synovial fluid.

A "sample collection apparatus," as used herein, refers to an apparatus that can be used for collection of a biological sample or into which a collected biological sample can be deposited or stored.

"Not in fluid contact," as used herein, indicates that fluid will not flow passively from the sample collection apparatus onto/into application point. For example, physical separation or separation by a physical component can be used.

### II. Point-of-Care Assay

A rapid, reliable, sensitive, qualitative, and quantitative point-of-care assay was developed to quantitatively measure small analytes, such as hormones, heavy metals, drugs, or drug metabolites, in a biological sample from a patient, including human and veterinary subjects. The point-of-care assay can be used in combination with binding agents and capture agents that specifically bind drug, drug metabolites, heavy metals, or hormones.

There may be specialized examples where aptamers (employed alone) have been shown to recognize small molecules, however, in general, the binding affinity is poor and ability to evolve aptamers against all small molecules targets of interest has proved elusive. Jayasena, Clin. Chem., 45:1628-50 (1999). This is likely due to the relative lack of cooperative binding opportunities presented in small molecule targets and aptamers lack the more complex binding pocket of antibodies. Antibodies on the other hand, have a much richer structural pocket to evolve binding based on hydrophobic, ionic, and steric interactions, but however, present with problems of cross-reactivity, especially where small molecules are concerned. The problem of cross-reactivity in antibodies is apparent when looking at the opiate structures since molecules are so structurally similar (differing by as little as one side group). Since antibodies are typically selected by the host organism immune system to bind with the highest affinity and this often times results in antibodies targeting structurally similar motifs. Hence the observed problems of cross reactivity observed in opiates. Additionally, raising antibodies *in vivo* against the desired immunocomplex is very difficult and impractical in almost all cases. In contrast, aptamers can be evolved against the target immunocomplexes under nearly identical conditions for the ultimate immunoassay.

Aptamers for proteins generally exhibit higher affinities, because of the presence of larger complex areas with structures rich in hydrogen-bond donors and acceptors. Affinities in the nanomolar and subnanomolar range have been measured for aptamers against different proteins. Mascini, et al. Angew. Chem. Int. Ed., 51:1316-1332 (2012). While not being bound by theory, the sandwich assays described herein "converts" small molecules which are in general poor targets for aptamers into proteins targets which are much better (i.e. nanomolar affinities compared to micromolar) targets. An immunocomplex of antibody and target molecule for example, represent a much richer target for aptamer binding. Evolving aptamers against the much richer binding target of the immunocomplex between antibodies and the target molecules is a much more generalizable strategy (i.e. no special label required for each target molecule for immobilization as the antibodies already present a generalizable handle for the required immobilization). So tight binders to the complex are much easier to evolve and in the case where the structurally similar motif is buried in the antibody pocket the external facing part of the molecule will likely contain the differentiating side group structure which can be recognized by the aptamer and hence lead to specific recognition of the desired immunocomplex as opposed to cross reactive immunocomplexes.

The point-of-care assay described herein is preferably a lateral flow immunoassay. In some embodiments, the assay involves the use of a sample collection apparatus that is not in fluid contact with the solid phase apparatus.

#### A. Small Analytes to be Detected

Analytes which can be detected using the point-of-care assay described herein include, but are not limited to drugs, or drug metabolites, hormones, and heavy metals.

##### i. Drugs and Drug Metabolites

The assay can be used to quantitatively determine the levels of drugs, for example, drugs with potential for abuse, in a biological sample. Exemplary drugs and drug metabolites are described below. In some embodiments the assay is semi-quantitative. For example a test strip where the different opiates and metabolites are indicated by separate

colors and analyzed by visual inspection.

**1. Opioids**

Exemplary opioids that can be detected using the quantitative point-of-care assay include morphine, codeine, thebaine, heroin, hydromorphone, hydrocodone, oxycodone, oxymorphone, desomorphine, nicomorphine, propoxyphene, dipropanoylmorphine, benzylmorphine, ethylmorphine, buprenorphine, fentanyl, pethidine, meperidine, methadone, tramadol, dextropropoxyphene, or analogues or derivatives thereof. For example, oxycodone (OxyContin®) is an opioid analgesic medication synthesized from opium-derived thebaine. Percocet is a combination of oxycodone and acetaminophen (paracetamol). Vicodin is a combination of hydrocodone and acetaminophen (paracetamol). In preferred embodiments, the assay quantitatively measures oxycodone, hydrocodone, or a combination thereof.

Exemplary opioid metabolites that can be detected using the disclosed quantitative lateral flow immunoassay are shown in Table 1.

Table 1. Opioid Metabolites

| Opioid | Key metabolizing enzyme(s) | Major metabolites |
|---|---|---|
| Buprenorphine | CYP3A4 | Norbuprenorphine, glucuronides |
| Codeine | CYP3A4, 2D6 | Morphine, glucuronides |
| Fentanyl | CYP3A4 | Norfentanyl |
| Hydrocodone | CYP3A4, 2D6 | Hydromorphone, norhydrocodone |
| Hydromorphone | UGT1A3, 2B7 | Glucuronides |
| Meperidine | CYP3A4, 2B6, 2C19 | Normeperidine |
| Methadone | CYP2B6 | EDDP |
| Morphine | UGT2B7 | Glucuronides |
| Oxycodone | CYP3A4, 2D6 | Noroxycodone, oxymorphone |
| Oxymorphone | UGT2B7 | 6-OH-oxymorphone, oxymorphone-3-glucuronide |
| Propoxyphene | CYP3A4 | Norpropoxyphene |
| Tramadol | CYP2D6 | O-desmethyl tramadol |

EDP=2-Ethyl-1,5-dimethyl-3,3-diphenylpyrrolinium.

**2. THC (Marijuana)**

In the cannabis plant, THC occurs mainly as tetrahydrocannabinol carboxylic acid (THC-COOH). Geranyl pyrophosphate and olivetolic acid react, catalyzed by an enzyme to produce cannabigerolic acid, which is cyclized by the enzyme THC acid synthase to give THC-COOH. Over time, or when heated, THC-COOH is decarboxylated producing THC. THC is metabolized mainly to 11-OH-THC (11-hydroxy-THC) by the human body. This metabolite is still psychoactive and is further oxidized to 11-Nor-9-carboxy-THC (THC-COOH). More than 100 metabolites n humans and animals can be identified, but 11-OH-THC and THC-COOH are the dominating metabolites. Metabolism occurs mainly in the liver by cytochrome P450 enzymes CYP2C9, CYP2C19, and CYP3A4. More than 55% of THC is excreted in the feces and approximately 20% in the urine. The main metabolite in urine is the ester of glucuronic acid and THC-COOH and free THC-COOH. In the feces, mainly 11-OH-THC is detected.

THC, 11-OH-THC and THC-COOH can be detected and quantified in blood, urine, hair, oral fluid or sweat. The concentrations obtained from such analyses can often be helpful in distinguishing active from passive use or prescription from illicit use, the route of administration (oral versus smoking), elapsed time since use and extent or duration of use.

**3. Nicotine**

As nicotine enters the body, it is distributed quickly through the bloodstream and crosses the blood-brain barrier reaching the brain within 10-20 seconds after inhalation. The elimination half-life of nicotine in the body is around two hours. The amount of nicotine absorbed by the body from smoking depends on many factors, including the types of tobacco, whether the smoke is inhaled, and whether a filter is used. For chewing tobacco, dipping tobacco, snus and snuff, which are held in the mouth between the lip and gum, or taken in the nose, the amount released into the body tends to be much greater than smoked tobacco.

Nicotine is metabolized in the liver by cytochrome P450 enzymes (mostly CYP2A6, and also by CYP2B6). A major metabolite of nicotine that is excreted in the urine is cotinine, which is a reliable and necessary indicator of nicotine

usage. Other primary metabolites include nicotine N'-oxide, nornicotine, nicotine isomethonium ion, 2-hydroxynicotine and nicotine glucuronide. Glucuronidation and oxidative metabolism of nicotine to cotinine are both inhibited by menthol, an additive to mentholated cigarettes, thus increasing the half-life of nicotine *in vivo.*

Nicotine (cotinine) can be quantified in blood, plasma, or urine to confirm a diagnosis of poisoning or to facilitate a medicolegal death investigation. Urinary or salivary cotinine concentrations are frequently measured for the purposes of pre-employment and health insurance medical screening programs. Careful interpretation of results is important, since passive exposure to cigarette smoke can result in significant accumulation of nicotine, followed by the appearance of its metabolites in various body fluids.

The CYP2A6 enzyme is genetically polymorphic with certain alleles predicting altered metabolic activity. As the primary enzyme for nicotine metabolism, variation in the metabolic activity of CYP2A6 has a significant effect on an individual's level of tobacco consumption. The reduced metabolism phenotype leads to higher blood/nicotine levels and smokers tend to compensate for this by smoking less. Conversely, individuals with increased metabolic rate tend to smoke more. Lower nicotine metabolism with CYP2A6 variants also has an effect on smoking cessation, with slow metabolizers demonstrating higher levels of cessation in transdermal nicotine therapy trials. This may be due to the higher therapeutic doses of nicotine that the slow metabolizer sub-group obtains from comparable levels of transdermal nicotine treatment. Normal metabolizers have lower cessation rates probably as a result of current treatments failing to provide high enough levels of replacement blood nicotine. These normal metabolizers may be candidates for higher-dose nicotine replacement, which might potentially give rise to adverse effects in those with impaired nicotine metabolism.

The disclosed compositions and methods may be used to evaluate a patient's metabolism of nicotine. For example, nicotine levels can be quantified using the disclosed compositions and methods after a controlled dosage of nicotine is administered to a patient. This can in some embodiments involve allowing the subject to smoke a cigarette. In preferred embodiments, a nicotine patch or gum is given to the subject for a prescribed amount of time. The amount of nicotine or a metabolite thereof (e.g., cotinine) in a biological sample of the subject may then be monitored for rate of change.

**ii. Hormones for Detection of Pregnancy or Time of Ovulation in Animals**

Unlike in humans, the hormonal cycles of domestic pets such as dogs and of livestock such as horses, cattle and swine are not as easily assayed and there are no point-of-care assays available. However, the reproductive levels of hormones indicating onset of ovulation, timing of breeding, and pregnancy are well understood by those skilled in the art and can be readily quantitated using a point of care immunoassay as described herein.

There are multiple hormones that help to regulate the estrus (heat) cycle and pregnancy in animals. These include estrogen, which stimulates the ovaries to produce eggs, luteinizing hormone (LH), which stimulates the ovaries to release the eggs, and progesterone, which maintains a pregnancy. Most mammals ovulate when the estrogen level in the blood is increasing. Dogs, however, ovulate when the estrogen level is declining and the progesterone level is increasing. Progesterone levels and luteinizing hormone (LH) levels are the best indicators of when ovulation will take place and when is the best time to breed.

**iii. Heavy Metal Ions**

Heavy metals are toxic and persistent environmental contaminants. Unlike carbon-based contaminants that can be completely degraded to relatively harmless products, metal ions can be transformed in only a limited number of ways by biological or chemical remediation processes.

Heavy metals have a specific gravity that is at least five times the specific gravity of water. Some well-known toxic metallic elements with a specific gravity that is five or more times that of water are arsenic, cadmium, iron, lead, and mercury. Additional toxic heavy metals include antimony, bismuth, cerium, chromium, cobalt, copper, gallium, gold, manganese, nickel, platinum, silver, tellurium, thallium, tin, uranium, vanadium, and zinc.

Heavy metal toxicity can result in damaged or reduced mental and central nervous function, lower energy levels, and damage to blood composition, lungs, kidneys, liver, and other vital organs. Long-term exposure may result in slowly progressing physical, muscular, and neurological degenerative processes that mimic Alzheimer's disease, Parkinson's disease, muscular dystrophy, and multiple sclerosis. Allergies are not uncommon and repeated long-term contact with some metals or their compounds may even cause cancer.

Small amounts of these elements are common in our environment and diet and are actually necessary for good health, but large amounts of any of them may cause acute or chronic toxicity. Heavy metals become toxic when they are not metabolized by the body and accumulate in the soft tissues. Heavy metals may enter the human body through food, water, air, or absorption through the skin when they come in contact with humans in agriculture and in manufacturing, pharmaceutical, industrial, or residential settings. Industrial exposure accounts for a common route of exposure for adults. Ingestion is the most common route of exposure in children. Children may develop toxic levels from the normal

hand-to-mouth activity of small children who come in contact with contaminated soil or by actually eating objects that are not food (dirt or paint chips). Less common routes of exposure are during a radiological procedure, from inappropriate dosing or monitoring during intravenous (parenteral) nutrition, from a broken thermometer, or from a suicide or homicide attempt.

For some heavy metals, toxic levels can be just above the background concentrations naturally found in nature. Therefore, it is important to take protective measures against excessive exposure. For persons who suspect that they or someone in their household might have heavy metal toxicity, testing is essential. The most common methods employed are flame atomic absorption spectrometry (AAS), graphite furnace atomic absorption spectrometry (GFAAS), anode stripping voltametry (ASV), inductively coupled plasma-atomic emission spectroscopy (ICP/AES), and inductively coupled plasma mass spectrometry (ICP/MS). However, these laboratory methods are labor-intensive, time-consuming, and expensive.

Antibody-based assays offer an alternate approach for metal ion detection. Immunoassays are quick, inexpensive, simple to perform, and reasonably portable; they can also be highly sensitive and selective. Sample analysis is one of the major costs in the remediation of a contaminated site, and studies have shown that the use of antibody-based assays can reduce analysis costs by 50% or more.

### B. Binding Agents and Capture Reagents

Binding agents for use in the disclosed assays include any molecule that selectively binds opioid analytes or calibration analytes. In preferred embodiments, the binding agents are antibodies, such as monoclonal antibodies, or aptamers, such as nucleic acid or peptide aptamers.

#### i. Antibodies

Antibodies that can be used in the compositions and methods include whole immunoglobulin (i.e., an intact antibody) of any class, fragments thereof, and synthetic proteins containing at least the antigen binding variable domain of an antibody. The variable domains differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen binding site of antibodies. Therefore, the disclosed antibodies contain at least the CDRs necessary to maintain DNA binding and/or interfere with DNA repair.

Fragments of antibodies which have bioactivity can also be used. The fragments, whether attached to other sequences or not, include insertions, deletions, substitutions, or other selected modifications of particular regions or specific amino acids residues, provided the activity of the fragment is not significantly altered or impaired compared to the nonmodified antibody or antibody fragment.

Techniques can also be adapted for the production of single-chain antibodies specific to an antigenic protein of the present disclosure. Methods for the production of single-chain antibodies are well known to those of skill in the art. A single chain antibody can be created by fusing together the variable domains of the heavy and light chains using a short peptide linker, thereby reconstituting an antigen binding site on a single molecule. Single-chain antibody variable fragments (scFvs) in which the C-terminus of one variable domain is tethered to the N-terminus of the other variable domain via a 15 to 25 amino acid peptide or linker have been developed without significantly disrupting antigen binding or specificity of the binding. The linker is chosen to permit the heavy chain and light chain to bind together in their proper conformational orientation.

Divalent single-chain variable fragments (di-scFvs) can be engineered by linking two scFvs. This can be done by producing a single peptide chain with two VH and two VL regions, yielding tandem scFvs. ScFvs can also be designed with linker peptides that are too short for the two variable regions to fold together (about five amino acids), forcing scFvs to dimerize. This type is known as diabodies. Diabodies have been shown to have dissociation constants up to 40-fold lower than corresponding scFvs, meaning that they have a much higher affinity to their target. Still shorter linkers (one or two amino acids) lead to the formation of trimers (triabodies or tribodies). Tetrabodies have also been produced. They exhibit an even higher affinity to their targets than diabodies.

Suitable antibodies may be commercially available. For example, antibodies that specifically bind codeine (abcam® #ab31202), heroin (Randox Life Sciences #PAS10133), morphine (abcam® #ab1060, #ab23357), hydrocodone (Abbiotec™ #252375), hydromorphone (abcam® #ab58932), oxycodone (abcam® #ab30544), propoxyphene

(abcam® #ab50726), buprenorphine (abcam® #ab31201), fentanyl (abcam® #ab30729, #ab31323), pethidine (Novus Biologicals ® #NBP1-41034), meperidine (abcam® #ab59530), methadone (abcam® #ab35799), and tramadol (abcam® #ab58934) are commercially available.

Several antibodies have been reported with the ability to bind heavy metals. Monoclonal antibodies directed toward mercuric ions have been generated by immunization of animals with a glutathione-Hg derivative (Wylie et al., Proc. Natl. Acad. Sci. USA 89:4104-4108 (1992)). Recombinant antibody fragments that preferentially recognized certain metals in complex with iminodiacetic acid have also been reported (Barbas et al., Proc. Natl. Acad. Sci. USA 90:6385-6389 (1993)). Monoclonal antibodies specific for complexes of EDTA-Cd(II), DTPA-Co(II), 2,9-dicarboxyl-1,10-phenanthroline-U(VI), or cyclohexyl-DTPA-Pb(II) have been used in competitive immunoassays for detecting chelated cadmium, lead, cobalt, and uranium (Blake DA, et al. Biosensors & Bioelectronics 16:799-809 (2001)).

Antibodies that specifically bind an analyte can also be made using routine methods. For example, antibodies can be purified from animals immunized with analyte. Monoclonal antibodies can be produced by fusing myeloma cells with the spleen cells from a mouse that has been immunized with the opioid analyte or with lymphocytes that were immunized *in vitro.* Antibodies can also be produced using recombinant technology.

The capture agent of the disclosed compositions and methods may be an antibody, such as an anti-metatype antibody. Anti-metatype antibodies are immunological reagents specific for the conformation of the liganded antibody active site which do not interact with bound ligand or unliganded antibody. An antibody that selectively binds a capture complex but not to free analyte may be obtained using standard methods known in the art. For example, a naive scFv antibody fragment phage display library may be used to select antibodies that bind to an immunocomplex of analyte and Fab fragments of antibodies that specifically bind the analyte. First the phages are preincubated to sort out those binding to Fab fragments as such. The unbound phages are separated and incubated with a mixture of analyte and immobilized Fab to select the phages that bind to the immunocomplex formed between the immobilized Fab and analyte. Unbound phages are washed away, and then those bound to the complex are eluted. The background is monitored by checking the binding to Fab in the absence of analyte. After several panning rounds a number of clones are picked up, sequenced and expressed resulting in an scFv fragment for use as a capture agent.

### ii. Nucleic Acid Aptamers

Nucleic acid aptamers are typically oligonucleotides ranging from 15-50 bases in length that fold into defined secondary and tertiary structures, such as stem-loops or G-quartets. The oligonucleotide may be DNA or RNA, and may be modified for stability. A nucleic acid aptamer generally has higher specificity and affinity to a target molecule than an antibody. Nucleic acid aptamers preferably bind the target molecule with a $K_d$ less than $10^{-6}$, $10^{-8}$, $10^{-10}$, or $10^{-12}$. Nucleic acid aptamers can also bind the target molecule with a very high degree of specificity. It is preferred that the nucleic acid aptamers have a $K_d$ with the target molecule at least 10, 100, 1000, 10,000, or 100,000 fold lower than the $K_d$ with other molecules. In addition, the number of target amino acid residues necessary for aptamer binding may be smaller than that of an antibody.

Nucleic acid aptamers are typically isolated from complex libraries of synthetic oligonucleotides by an iterative process of adsorption, recovery and reamplification. For example, nucleic acid aptamers may be prepared using the SELEX (Systematic Evolution of Ligands by Exponential Enrichment) method. The SELEX method involves selecting an RNA molecule bound to a target molecule from an RNA pool composed of RNA molecules each having random sequence regions and primer-binding regions at both ends thereof, amplifying the recovered RNA molecule via RT-PCR, performing transcription using the obtained cDNA molecule as a template, and using the resultant as an RNA pool for the subsequent procedure. Such procedure is repeated several times to several tens of times to select RNA with a stronger ability to bind to a target molecule. The base sequence lengths of the random sequence region and the primer binding region are not particularly limited. In general, the random sequence region contains about 20 to 80 bases and the primer binding region contains about 15 to 40 bases. Specificity to a target molecule may be enhanced by prospectively mixing molecules similar to the target molecule with RNA pools and using a pool containing RNA molecules that did not bind to the molecule of interest. An RNA molecule that was obtained as a final product by such technique is used as an RNA aptamer. Representative examples of how to make and use aptamers to bind a variety of different target molecules can be found in U.S. Patent Nos. 5,476,766, 5,503,978, 5,631,146, 5,731,424 , 5,780,228, 5,792,613, 5,795,721, 5,846,713, 5,858,660, 5,861,254, 5,864,026, 5,869,641, 5,958,691, 6,001,988, 6,011,020, 6,013,443, 6,020,130, 6,028,186, 6,030,776, and 6,051,698. An aptamer database containing comprehensive sequence information on aptamers and unnatural ribozymes that have been generated by *in vitro* selection methods is available at aptamer.icmb.utexas.edu.

In some embodiments, the aptamer is a molecular aptamer beacon. A molecular beacon is a hairpin-shaped oligonucleotide with a fluorophore and a quencher linked to each end of its stem. The signal transduction mechanism for molecular recognition is based on resonance fluorescence energy transfer (FRET) and the conformational change of a molecular beacon. The molecular beacon acts like a switch that is normally closed to bring the fluorophore/ quencher

pair together to turn fluorescence "off". When binding to a target biomolecule, it undergoes a conformational change that opens the hairpin structure and separates the fluorophore and the quencher, thus turning "on" the fluorescence. Molecular aptamer beacons were developed to combine the sequence specificity and sensitivity of aptamers with the real-time detection advantages of molecular beacons. Briefly, oligonucleotides containing a nucleic acid aptamer sequence are designed to have complementary DNA or RNA sequences that form a hairpin, which is opened when the aptamer sequence binds its target. Molecular aptamer beacons are described in Cho et al. Annu Rev Anal Chem (Palo Alto Calif) 2:241-64 (2009), Hamaguchi N, et al. Anal Biochem. 294(2):126-31 (2001); Li JJ, et al. Biochem Biophys Res Commun. 292(1):31-40 (2002).

**iii. Peptide Aptamers**

Peptide aptamers are small peptides with a randomized amino acid sequence that are selected for their ability to bind a target molecule. Peptide aptamer selection can be made using different systems, but the most used is currently the yeast two-hybrid system. Peptide aptamer can also be selected from combinatorial peptide libraries constructed by phage display and other surface display technologies such as mRNA display, ribosome display, bacterial display and yeast display. These experimental procedures are also known as biopannings. Among peptides obtained from biopannings, mimotopes can be considered as a kind of peptide aptamers. All the peptides panned from combinatorial peptide libraries have been stored in a special database with the name MimoDB.

**C. Biological Sample**

In the disclosed assays, a biological sample is assessed for the presence, absence, or most preferably, the quantity of a small analyte. The biological sample is preferably a bodily fluid, such as whole blood, plasma, serum, urine, cerebrospinal fluid, saliva, semen, vitreous fluid, or synovial fluid. In a preferred embodiment, the bodily fluid is whole blood, plasma, or serum.

*Assay Fluid*

An aqueous assay fluid can also be introduced to the biological sample, forming a mixed fluid sample. The assay fluid supports a reaction between the analyte and the labeled binding agent (e.g., does not interfere with binding) and has a viscosity that is sufficiently low to allow movement of the assay fluid by capillary action. In some embodiments, the assay fluid contains one or more of the following components: a buffering agent (e.g., phosphate); a salt (e.g., NaCl); a protein stabilizer (e.g., bovine serum albumin "BSA", casein, serum); and a detergent such as a nonionic detergent or a surfactant (e.g., NINATE® 411, ZONYL® FSN 100, AEROSOL OT 100%, GEROPON® T-77, BIO-TERGE® AS-40, STANDAPOL® ES-1, TETRONIC® 1307, SURFNYOL® 465, SURFYNOL® 485, SURFYNOL® 104PG-50, IGEPAL® CA210, TRITON™ X-45, TRITON™ X-100, TRITON™ X305, SILWET® L7600, RHODASURF® ON-870, CREMOPHOR® EL, TWEEN® 20, TWEEN® 80, BRIJ 35, CHEMAL LA-9, PLURONIC® L64, SURFACTANT 10G, SPAN™ 60). Optionally, if desired, the assay fluid can contain a thickening agent. Representative assay fluids include saline, or 50 mM Tris-HCl, pH 7.2. In some embodiments, the assay fluid is water.

**D. Lateral Flow Device**

In preferred embodiments, the disclosed point-of-care assay is a lateral flow assay, which is a form of immunoassay in which the test sample flows along a solid substrate via capillary action. As illustrated in Figure 4, a lateral flow device **10** includes a solid substrate **12,** such as a membrane strip, having an application point **14,** an optional conjugate zone **16,** a capture zone **18,** and an absorbent zone **20** (e.g., a wicking pad). Binding agents are optionally present in the conjugate zone **16.** Capture agents are immobilized in the capture zone **18,** which preferably contains a plurality of capture lines **22** for detecting captured analyte (capture complex).

**i. Solid Substrate**

The solid substrate **12,** such as a membrane strip, can be made of a substance of sufficient porosity to allow movement of antibodies and analyte by capillary action along its surface and through its interior. Examples of suitable membrane substances include: cellulose, cellulose nitrate, cellulose acetate, glass fiber, nylon, polyelectrolyte ion exchange membrane, acrylic copolymer/nylon, and polyethersulfone. In a one embodiment, the membrane strip is made of cellulose nitrate (e.g., a cellulose nitrate membrane with a Mylar backing) or of glass fiber.

In a preferred embodiment, the membrane strip is FUSION 5™ material (Whatman), which is a single layer matrix material that performs all of the functions of a lateral flow strip. For FUSION 5™, the optimal bead size is approximately 2 microns; the FUSION 5™ material has a 98% retention efficiency for beads of approximately 2.5 microns. Beads of 2.5 microns will not generally enter the matrix, whereas beads of below 1.5 microns will be washed out of the matrix.

**ii. Application point**

The solid substrate **12** includes an application point **14,** which can optionally include an application pad. For example, if the sample containing the analyte contains particles or components that should preferentially be excluded from the immunoassay, an application pad can be used. The application pad typically can filter out particles or components that are larger (e.g., greater than approximately 2 to 5 microns) than the particles used in the disclosed methods. The application pad may be used to modify the biological sample, e.g., adjust pH, filtering out solid components, separate whole blood constituents, and adsorb out unwanted antibodies. If an application pad is used, it rests on the membrane, immediately adjacent to or covering the application point. The application pad can be made of an absorbent substance which can deliver a fluid sample, when applied to the pad, to the application point on the membrane. Representative substances include cellulose, cellulose nitrate, cellulose acetate, nylon, polyelectrolyte ion exchange membrane, acrylic copolymer/nylon, polyethersulfone, or glass fibers. In one embodiment, the pad is a Hemasep™-V pad (Pall Corporation). In another embodiment, the pad is a Pall™ 133, Pall™ A/D, or glass fiber pad.

**iii. Conjugate Zone**

The solid substrate **12** optionally contains a conjugate zone **16,** which contains binding agents. In some embodiments, the conjugate zone contains binding agents which bind the analyte to be measured and a control analyte. When the sample migrates through the conjugate zone containing binding agents, the analytes in the sample interacts with the binding agents to form capture complexes.

**iv. Absorbent Zone**

The absorbent zone **20** preferably contains a wicking pad. If a wicking pad is present, it can similarly be made from such absorbent substances as are described for an application pad. In a preferred embodiment, a wicking pad allows continuation of the flow of liquid by capillary action past the capture zones and facilitates the movement of non-bound agents away from the capture zones.

**v. Capture Zone**

The capture zone **18** contains capture agent immobilized (e.g., coated on and/or permeated through the membrane) to the membrane strip. In preferred embodiments, the capture agent is conjugated to a capture particle that is immobilized in the capture zone **18.**

The capture zone **18** is preferably organized into one or more capture lines containing capture agents. In preferred embodiments, the capture zone contains a plurality of capture lines for multiplex analysis, i.e., detection of two or more analytes. In addition, the capture zone **18** may contain one or more control capture lines for detecting the presence of control analyte (i.e., control or calibration capture zone). In preferred embodiments the control analyte is a compound that is not normally present in any prescription or non-prescription drug, food, beverage, or supplement. Preferably, the control analyte capture reagent specifically binds the control analyte but does not interact with the sample analyte being measured.

The calibration capture zone is preferably positioned such that the sample capture zone is between the application point and the calibration capture zone. In a preferred embodiment, the calibration capture zone is closely adjacent to the sample capture zone, so that the dynamics of the capillary action of the components of the assay are similar (e.g., essentially the same) at both the calibration capture zone and the sample capture zone. For example, the two capture zones are sufficiently close together such that the speed of the liquid flow is similar over both zones. Although they are closely adjacent, the calibration capture zone and the sample capture zone are also sufficiently spaced such that the particles arrested in each zone can be quantitated individually (e.g., without cross-talk). Furthermore, in a preferred embodiment, the sample capture zone is separated from the application point by a space that is a large distance, relative to the small distance between the sample capture zone and the calibration capture zone. Because particle capture is a rate limiting step in the assay, the distance between the application point and the capture zones (where particles are captured) must be sufficient to retard the speed of the liquid flow to a rate that is slow enough to allow capture of particles when the liquid flow moves over the sample capture zone. The optimal distances between the components on the membrane strip can be determined and adjusted using routine experimentation

In some embodiments, the capture zone **18** contains at least one capture line **22** with capture agents for

detecting a dilution control analyte, i.e., an analyte that is typically present in the biological sample at predictable concentrations. Creatine is a particularly preferred dilution control analyte when the biological sample is urine. The typical human reference ranges for serum creatinine are 0.5 to 1.0 mg/dL (about 45-90 μmol/L) for women and 0.7 to 1.2 mg/dL (60-110 μmol/L) for men.

In some embodiments, the capture zone **18** contains one or more capture lines with capture agents for detecting reference analytes. The reference analytes may be administered to the biological sample at known concentrations. These reference values can facilitate quantitative correlations between label detection and analyte amounts.

### vi. Capture Particles

Capture particles are particles, such as polymeric particles, which can be coated with the capture agent and immobilized to the membrane in the capture zone **18.** In preferred embodiments, the particles are physically trapped within the membrane. This allows for selection of optimal particle chemistry that is not influenced by the need for chemical immobilization. Suitable capture particles include liposomes, colloidal gold, organic polymer latex particles, inorganic fluorescent particles, and phosphorescent particles. In some embodiments, the particles are polystyrene latex beads, and most particularly, polystyrene latex beads that have been prepared in the absence of surfactant, such as surfactant-free Superactive Uniform Aldehyde/Sulfate Latexes (Interfacial Dynamics Corp., Portland, Oreg.).

In preferred embodiments, the particles are monodisperse polymer microspheres based on melamine resin (MF) (e.g., available from Sigma-Aldrich). Melamine resin microspheres are manufactured by acid-catalyzed hydrothermal polycondensation of methylol melamine in the temperature range of 70-100 °C without any surfactants. Unmodified MF particles have a hydrophilic, charged surface due to the high density of polar triazine-amino and -imino groups. The surface functional groups (methylol groups, amino groups, etc.) allow covalent attachment of other ligands. For special applications, the MF particles can be modified by incorporation of other functionalities such as carboxyl groups. This increases possible surface derivatization such as chromophore or fluorophore labeling.

The particles can be labeled to facilitate detection by a means which does not significantly affect the physical properties of the particles. For example, the particles can be labeled internally (that is, the label is included within the particle, such as within the liposome or inside the polystyrene latex bead). Representative labels include luminescent labels; chemiluminescent labels; phosphorescent labels; fluorescent labels; phosphorescent labels; enzyme-linked labels; chemical labels, such as electroactive agents (e.g., ferrocyanide); and colorimetric labels, such as dyes. In one embodiment, a fluorescent label is used. In another embodiment, phosphorescent particles are used, particularly up-converting phosphorescent particles, such as those described in U.S. Patent No. 5,043,265.

The particles are preferably coated with capture agent, such as a sample analyte capture agent and control analyte capture agent. They can be prepared by mixing the capture agent in a conjugation buffer. A covalent coupling onto the particles is then performed, resulting in random binding of the capture agents onto the particle.

## E. Sample Collection Apparatus

The quantitative point-of-care assay may involve the use of a sample collection apparatus that is not in fluid contact with the solid phase apparatus. The sample collection apparatus can be any apparatus which can contain binding agents and to which a measured volume of fluid sample can be added. Representative sample collection apparatus include a sample tube, a test tube, a vial, a pipette or pipette tip, a syringe. In a preferred embodiment, the sample collection apparatus is a pipette or pipette tip.

In one embodiment, the sample collection apparatus contains a population of binding agents. The binding agents can be stored within the sample collection apparatus in a stable form, i.e., a form in which the agents do not significantly change in chemical makeup or physical state during storage. The stable form can be a liquid, gel, or solid form. In preferred embodiments, the agents are evaporatively dried; freeze-dried; and/or vacuum-dried. In one preferred embodiment, the sample collection apparatus contains a pipette tip having vacuum-dried binding particles within its tip. In another preferred embodiment, the sample collection apparatus contains a pipette tip having vacuum-dried analyte binding particles and vacuum-dried calibration analyte binding particles within its tip.

In other embodiments, the sample collection apparatus contains a population of drug binding particles and a population of calibration binding particles. The sample collection apparatus may also contain calibration analyte. If so, the population of particles is located at a different place in the sample collection apparatus from the calibration analyte. The calibration analyte can also be evaporatively-dried, vacuum-dried or freeze-dried in the sample collection apparatus. If the calibration analyte is not stored within the sample collection apparatus, then it can be present in the assay fluid.

In either embodiment, the population of particles varies, depending on the size and composition of the particles, the composition of the membrane of the solid phase apparatus, and the level of sensitivity of the assay. The population typically ranges approximately between 1 x $10^3$ and 1 x $10^9$, although fewer or more can be used if desired. In certain embodiments the amount of particles is determined as an amount of solids in the suspension used to apply the particles

for storage within the sample collection apparatus. For example, when applying the particles in solution for freeze- or vacuum-drying in the sample collection apparatus, a suspension of approximately 0.05% to 0.228% solids (W/V) in 5 μl of suspension can be used. Alternatively, other amounts can be used, including, for example, from approximately 0.01% to 0.5% (W/V).

The binding particles (coated with both drug binding agent and calibration binding agent), or the analyte binding particles and the calibration analyte binding particles, can be stored within the sample collection apparatus in a stable form, i.e., a form in which the particles do not significantly change in chemical makeup or physical state during storage. The analyte binding particles and the calibration analyte binding particles are stored at the same location within the sample collection apparatus (e.g., applied as a homogeneous mixture to the location).

### III. Assay Method

The lateral flow assay can be used to detect a small analyte, such as drug, drug metabolite, heavy metal, or hormone, in a biological sample. The assay generally involves combining the biological sample with an assay fluid, a drug binding agent that specifically binds a drug analyte, a calibration/control analyte, and a calibration/control binding agent that specifically binds the calibration analyte. Contacted capture particles may or may not have analyte bound to the analyte binding agent, depending on whether or not analyte is present in the fluid sample and whether analyte has bound to the analyte binding agent on the binding particles. Because there are multiple binding sites for analyte on the capture particles, the presence and the concentration of analyte bound to particles varies; the concentration of analyte bound to the particles increases proportionally with the amount of analyte present in the fluid sample, and the probability of a particle being arrested in the sample capture zone similarly increases with increasing amount of analyte bound to the drug binding agent on the particles. Thus, the population of contacted binding particles may contain particles having various amount of analyte bound to the drug binding agent, as well as particles having no analyte bound to the drug binding agent. In some preferred embodiments, only the mobile element contains a label.

In a preferred embodiment, the drug analyte and the control analyte have similar physical properties. For example, the control analyte is preferably a small molecule of similar size to the drug analyte of interest. However, the calibration analyte is preferably not present in human biological samples and does not cross-react with the drug binding agent. Therefore, in preferred embodiments, the calibration analyte is a compound that is not normally present in any prescription or non-prescription drug, food, beverage, or supplement.

In another preferred embodiment, the drug binding agent and the control binding agent also have similar properties. For example, if the drug binding agent is an antibody, the calibration binding agent is also preferably an antibody. Moreover, the affinity and/or avidity of the calibration/control binding agent for the calibration/control analyte is preferably comparable (e.g., within one order of magnitude) to the affinity and/or avidity of the drug binding agent for the drug analyte.

#### A. Sample Preparation

In one embodiment, the biological sample is first combined with a binding agent in an assay fluid to produce a mixed fluid sample. If analyte is present in the mixed fluid sample, binding occurs between the analyte and the binding agent to produce capture complex. The degree of binding increases as the time factor of the conditions increases. While the majority of binding occurs within one minute, additional incubation for more than one minute, 2 minutes, 5 minutes, 10 minutes, or 15 minutes results in additional binding. In some embodiments, the binding agent is present in the sample collection apparatus. The biological sample is preferably mixed with calibration analyte and particles coated with a calibration binding agent. In preferred embodiments, the binding particles contain detectable labels.

If there is no calibration analyte in the sample collection apparatus, then the assay fluid can contain calibration analyte. Therefore, the mixed fluid sample contains drug binding particles, calibration binding particles, calibration analyte and sample analytes (if present).

In still other embodiments, the binding agents are present in the conjugation zone of the lateral flow membrane strip. In these embodiments, the sample is collected into any sample collection container used in the art to collect such samples, for example, any common laboratory container for collecting random urine samples can be used to collect urine. Samples should be collected following recommended guideline known in the art to avoid false negative results as described with respect to urine samples for example in Moeller, et al., Mayo Clin. Proc. 83(1):66-76 (2008).

#### B. Application of Sample

The sample is applied to the application point **14** of the membrane strip, or to the application pad, if present. After the membrane strip is contacted with the sample, the membrane strip is maintained under conditions (e.g., sufficient time and fluid volume) which allow the labeled binding agents to move by capillary action along the membrane to and

through the capture zone **18** and subsequently beyond the capture zones **18** (e.g., into a wicking pad), thereby removing any non-bound labeled binding agents from the capture zones. In some embodiments, the sample migrates through the conjugate zone containing binding agents. The analyte in the sample interacts with the binding agents to form capture complexes.

As the applied sample passed through the membrane strip, analyte bound (sample/control analyte) to binding agent (capture complex) are immobilized by capture agents in the capture zone **18,** which are preferably conjugated to immobilized capture particles. The capture zone **18** is preferably organized into one or more capture lines in specific areas of the capture zone where they serve to capture the capture complexes as they migrate by the capture lines. The capture zone **18** preferably contains a plurality of capture lines **22** for multiplex analysis and quantification.

Capillary action subsequently moves any binding agents that have not been arrested onwards beyond the capture zone **18,** for example, into a wicking pad which follows the capture **18** zone. If desired, a secondary wash step can be used. Assay fluid can be applied at the application point after the mixed fluid sample has soaked in to the membrane or into the application pad, if present. The secondary wash step can be used at any time thereafter, provided that it does not dilute the mixed fluid sample. A secondary wash step can contribute to reduction of background signal when the capture particles are detected.

### C. Detection

The amount of analyte bound by binding agents arrested in the capture zone (sandwich complex) may then be detected. The labeled binding or capture agents are preferably detected using an appropriate means for the type of label used. In a preferred embodiment, the labeled binding or capture agents are detected by an optical method, such as by measuring absorbance or fluorescence. In preferred embodiments, the particles are detected using an ESEQuant™ Lateral Flow Immunoassay Reader (Qiagen). Alternatively, labeled binding or capture agents can be detected using electrical conductivity or dielectric (capacitance). Alternatively, electrochemical detection of released electroactive agents, such as indium, bismuth, gallium or tellurium ions, or ferrocyanide can be used. For example, if liposomes are used, ferrocyanide encapsulated within the liposome can be released by addition of a drop of detergent at the capture zone, and the released ferrocyanide detected electrochemically. If chelating agent-protein conjugates are used to chelate metal ions, addition of a drop of acid at the capture zone will release the ions and allow quantitation by anodic stripping voltametry. Alternatively, magnetic particle detection methods as well as colorimetic methods can be utilized.

### D. Interpreting Results

For non-competitive assays, the amount of analyte in the sample is directly related to the level of detection agent detected in a capture line. This value is preferably normalized by the amount of another detectable label immobilized within the membrane (e.g., capture zone) to account for variations in detection device and parameters (e.g., light intensity). This normalized value may then be plotted against a standard curve or response surface that correlates these normalized values to analyte concentration. For example, a standard curve or response surface may be prepared in advance using analyte standards. In addition, three or more internal standard analytes may be detected in the assay and used to adjust or select the standard curve or surface from reference curves or surfaces.

The response surface methodology (RSM) is a collection of mathematical and statistical techniques useful for the modeling and analysis of problems in which a response of interest is influenced by several variables and the objective is to optimize this response (Montgomery, Douglas C. 2005. Design and Analysis of Experiments: Response surface method and designs. New Jersey: John Wiley and Sons, Inc.). In some cases, a fitted RSM model is used to more accurately determine the analyte concentration from a multiplexed assay with a range of detection agents. For example, the binding of analyte to the capture agent is dependent both on the particular agent $x_1$ (e.g., antibody) and the concentration of the analyte $x_2$ (e.g., THC). The test can be conducted with combinations of $x_1$ (continuous variable) and $x_2$ (cardinal variable) to determine a response to analyte values (continuous variable). The cardinal value can constitute the physical ordering on the test strip (e.g., line 1, line 2, etc...). However, as the RSM is fitted to minimize error and not intrinsically related to the actual physical ordering of the binding agents in the assay, other orderings (i.e. ordinal, continuous) may be preferred to simplify the fit. In the simplest case, the fluorescent intensity $y$ is the response variable, and this detected intensity is a function of analyte concentration ($x_1$) and the binding agent used ($x_2$). This function can be expressed as

$$y = f(x_1, x_2) + \varepsilon.$$

The variables $x_1$ and $x_2$ are independent variables where the response $y$ depends on them. Additional inde-

pendent variables (e.g., $x_3$, $x_4$, etc...) may also be used to improve quantitative results. The dependent variable *y* is a function of $x_1$, $x_2$, and the experimental error term, denoted as $\varepsilon$. The error term $\varepsilon$ represents any measurement error on the response, as well as other type of variations not counted in *f*. This is a statistical error that is assumed to be distributed normally with zero mean and variance $s^2$. In most RSM problems, the true response function *f* is unknown. In order to develop a proper approximation for *f*, the experimenter starts with a low-order polynomial in a small test region. If the response can be defined by a linear function of independent variables, then the approximating function is a first-order model. A first-order model with two independent variables can be expressed as

$$y = \beta_0 + \beta_1 x_1 + \beta_2 x_2 + \varepsilon.$$

If there is a curvature in the response surface, as is commonly the case with binding curves, then a higher degree polynomial should be used. The approximating function with two variables is called a second-order model:

$$y = \beta_0 + \beta_1 x_1 + \beta_2 x_2 + \beta_{11} x^2_{11} + \beta_{22} x^2_{22} + \beta_{12}\, x_1\, x_2 + \varepsilon$$

Higher order models are possible but in general all RSM problems use either one or a mixture of both of these models.

Given the RSM equation where *y* is the detected signal and the position of the signal is known to be associated with a particular binding agent $x_2$, then one can solve for the unknown concentration $x_1$, where $x_1$ is a positive real value.

In the preferred embodiment, the response value *y* is the normalized intensity. This normalization removes noise associated with variations in light intensity resulting from the light source (i.e. aging, warm up, low frequency drift, etc...). Fluorescence detection that is dependent upon analyte concentration (e.g., on binding agents or aptamer beacons) is preferably normalized against another fluorescence marker present in or on the membrane. For example, a fluorescent bead, optionally at the same excitation and emission wavelengths as the fluorescent label dependent upon analyte concentration, may be included in a separate control line to normalize the detection output. This can be represented by the formula

$$y_n = y_x / y_c,$$

where $y_x$ is the detected response of the unknown analyte, $y_c$ is the detected response of the control marker in or on the membrane, and $y_n$ is the normalized response.

In the preferred embodiment, the analyte concentration value $x_1$ is a dimensionless value scaled by the highest detection concentration for particular analyte associated with the respective binding agent. This simplifies the RSM fitting by putting the various detection analytes on similar scales although the diagnostically relevant detection ranges may be vastly different between the respective analytes (i.e. fentanyl vs. morphine). This can be represented by the formula

$$x_{1n} = x_1 / x_C,$$

where $x_{1n}$ is the dimensionless concentration of the unknown analyte, $x_1$ is the concentration of the analyte, and $x_C$ is the concentration of the highest level of analyte in the assay (i.e. higher levels not diagnostically relevant). To recover the actual value of analyte from the dimensionless value derived from the RSM, one just multiplies by the constant $x_C$ for that analyte. Typically this operation would be internal to the device operation and invisible to the end user that would just see a reported concentration for the detected analyte.

In the preferred embodiment, the binding agent $x_2$ is expressed as a continuous value by ordering the determined calibration curves for each analyte and binding agent and determining a value $x_2$ for each binding so as to give the simplest RSM with minimal error. The naive case would order these in a cardinal manner such that the lowest response curves were first and progressed to steeper responses. However, as the physical location of the detection lines are not related to the ordering for the determined surface, continuous values can be assigned to optimize the RSM fitting (i.e. morphine = 0.2, fetanyl = 1.1, etc...)

In the preferred embodiment, this optimized RSM surface determined by testing known combinations of analytes and binding agents is used to solve for unknown analyte concentrations. Inclusion of internal standards improves this calculation by ensuring that for a given test the determined values are within expected variances ($\varepsilon$) or if not can be adjusted to compensate and improve the accuracy of the individual test. In the simplest example, the included internal

standards may indicate an error associated with a constant offset, $\beta_0 + \beta'$, and can correct the results by subtracting the determined offset, $\beta'$, from the formulae to determine unknown values. Inclusion of three or more standards would allow more complex corrections to the RSM surface, including curvature corrections, without having to derive an entirely new model. Preferably five internal standard would be used and cover the four extremities of the RSM model plus a center point.

## IV. Kits

Kits for use in the disclosed methods are also provided. In one embodiment, the kit includes the lateral flow device disclosed herein, which optionally includes a conjugate zone **16,** which preferably comprises a binding agent. The kit optionally contains a sample collection apparatus.

In some embodiments the sample collection apparatus which is not in fluid contains with the lateral flow device. In some embodiments, the sample collection apparatus contains a population of binding agents which are preferably, evaporatively, freeze- or vacuum dried onto the sample collection apparatus. Kit components additionally can include: analytes at known concentrations for generating a standard curve, capture particles, particles and conjugation buffer for coating particles with binding agents, disposal apparatus (e.g., biohazard waste bags), and/or other information or instructions regarding the sample collection apparatus (e.g., lot information, expiration date, etc.).

## EXAMPLE 1: Aptamers Selection

The antibodies shown in Table I are useful in a proof of concept assay to identify aptamers that can be used in non-competitive assay for oxycodone. Hydromorphone can be used as a negative control. The antibodies all have cross reactivity to oxycodone, hydrocodone, oxymorphone, noroxycodone, and hydromorphone as shown in Table I. The structures of oxycodone, hydrocodone, oxymorphone, noroxycodone, and hydromorphone are shown below.

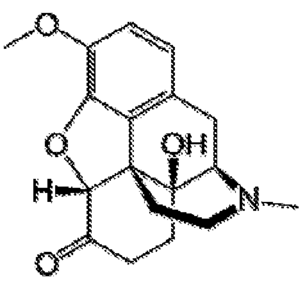

**Oxycodone**

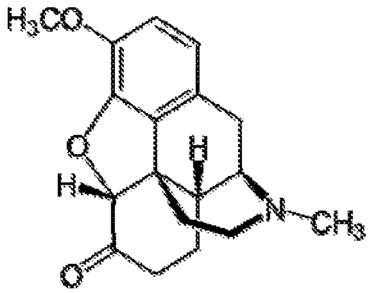

**Hydrocodone**

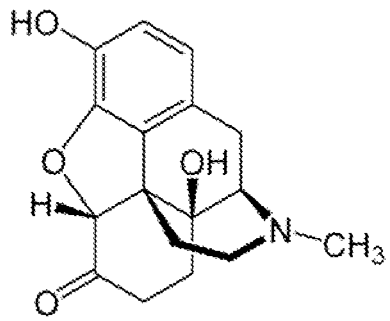

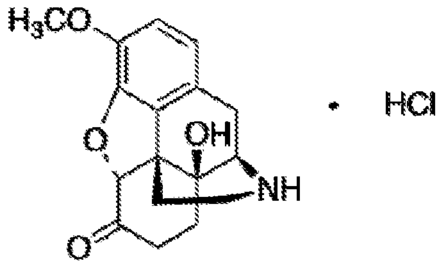

**Oxymorphone** **Noroxycodone**

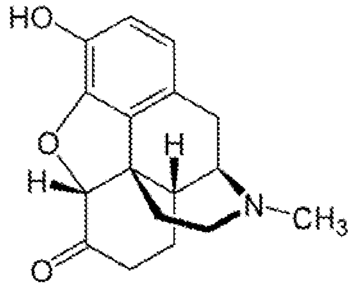

**Hydromorphone**

The antibodies used for proof of concept, and relative activity to oxycodone are shown in Table I.

**TABLE I: Antibodies and their relative activity to oxycodone**

| **Antibody/** | **Relative Activity to Oxycodone** | **Hydrocodone** | **Relative Activity to Oxymorphone** | **Noroxycodone** | **Hydromorphone** |
|---|---|---|---|---|---|
| **PAS9713** | 100 | 4.1 | 13.2 | 0.1 | 0.2 |
| **PAS9771** | 100 | 2282.4 | 4.4 | 0.1 | 163 |
| **PAS9712** | 100 | 34.3 | 0.1 | 19.3 | .0.1 |
| ***MBS315355** | 100 | 3.7 | 47.2 | ND | 0.7 |

*Source of antibody is rabbit. All other antibodies are raised in sheep

PAS9713, PAS9712 are sheep polyclonal antibody with oxycodone as its target, available from Randox Life Sciences. PAS9771 is an anti-hydromorphone antibody available from Randox Life Sciences. MBS315355 is an anti-oxycodone antibody raised in rabbit, avialbale from MyBioSource, San Diego, CA. Although PAS9713, PAS9712, and PAS9771 are anti-oxycodone antibodies, they cross react with hydrocodone, oxymorphone, and hyromorphone as shown in Table 1.

Aptamers selective for a drug of interest, for example, oxycodone, can be selected using the *in vitro* process, SELEX. Aptamers are selected based on their recognition of an oxycodone immunocomplex.

Briefly, the SELEX process begins with a large random oligonucleotide library (pool), whose complexity and diversity is dependent on the number of its random nucleotide positions. Mayer, Anew. Chem. Int. Ed., 48:2672-2689 (2009). During the SELEX procedure, binding DNA from the sequences are separated from DNA lacking affinity. This can be accomplished by immobilizing the target of interest, for example, the antibody-drug complex, to a column matrix, usually agarose or sepharose, and allowing easy partitioning of unwanted sequences through multiple washes. Alternatively, magnetic beads can be used as the solid matrix, as described for the FluMag SELEX system, Stoltenburg, et al., Anal. Bioanal. Chem. 383:83-91 (2005). This results in an enriched pool, which is subjected to further selection rounds that serve to increase the pool's affinity for the target molecule (positive selections) or eliminate members of the pool that have affinity for undesirable compounds (negative selections). After several rounds, the enriched pool is cloned, sequenced, and characterized to find aptamers which show selectivity for the drug of interest. Aptamer binding to antibody (without drug) can be used in a negative selection assay, to select out apatamers which show non-specific binding to antibodies. Negative selection for non-specific binding to immunocomplex (using hydromorphone for example,) can also be to enrich the aptamer pool selective to oxycodone.

## Claims

1. A non-competitive assay for quantitatively measuring the amount of an analyte in a biological sample from a subject, comprising:

   a) reacting the biological sample with

      (i) a binding agent that selectively binds the analyte to form a capture complex of the binding agent and analyte, wherein the binding agent is not an aptamer, and
      (ii) an aptamer that selectively binds to the analyte bound to the capture complex to form a complex of the binding agent, aptamer, and analyte, and

   b) measuring the amount of aptamer bound complex;

   wherein the analyte has a molecular weight of less than 2,000 Daltons, and the amount of complex formation is directly related to the amount of the analyte in the sample.

2. The non-competitive assay method of any one of claim 1, wherein

   a) the binding agent comprises an antibody; and/or
   b) the aptamer is a nucleic acid aptamer, or peptide aptamer that selectively binds the capture complex.

3. The non-competitive assay method of claim 1 or 2, wherein the binding agent is linked to a first detectable label and/or the aptamer is linked to a second detectable label, and optionally:

   a) wherein the first detectable label and the second detectable label are fluorescent molecules with distinct excitation and emission wavelength combinations;
   b) wherein the first detectable label and the second detectable label form a fluorescence resonance energy transfer (FRET) donor-acceptor pair; and/or
   c) wherein the nucleic acid aptamer binding agent comprises a fluorophore and quencher pair, wherein formation of the complex results in detectable quenching or unquenching of the flourophore.

4. The non-competitive assay method of any preceding claims, wherein the analyte is a hormone, drug, drug metabolite or heavy metal ion.

5. The non-competitive assay method of claim 4, wherein the drug is a drug of abuse, for example an opioid or opioid

metabolite, such as:

a) an opioid selected from the group consisting of morphine, codeine, thebaine, heroin, hydromorphone, hydrocodone, oxycodone, oxymorphone, desomorphine, nicomorphine, propoxyphene, dipropanoylmorphine, benzylmorphine, ethylmorphine, buprenorphine, fentanyl, pethidine, meperidine, methadone, tramadol, dextropropoxyphene, and combinations thereof.
b) oxycodone, hydrocodone, or a combination thereof; or
c) noroxycodone, oxymorphone, hydromorphone, norhydrocodone, or a combination thereof.

**6.** The non-competitive assay method of claim 5, wherein the drug is a drug of abuse and the analyte is THC or a metabolite thereof, or wherein the drug is an opioid or opioid metabolite and the analyte is nicotine or a metabolite thereof.

**7.** The non-competitive assay method of any one of claims 1 to 6, comprising a lateral flow immunoassay.

**8.** The non-competitive assay method of claim 7, comprising

a) optionally adding binding agents to the biological sample;
b) applying the biological sample to a membrane strip comprising an application point, an optional conjugation zone, a capture zone, and an absorbent zone, wherein the conjugation zone comprises the binding agent, wherein the capture zone comprises the aptamer immobilized in or on the membrane strip, wherein the biological sample is applied to the application point;
c) optionally maintaining the membrane strip under conditions that allow analyte present in the biological sample to move by capillary action through the membrane strip to the conjugation zone and to allow binding of the binding agent to the analyte to form a capture complex;
d) maintaining the membrane strip under conditions that allow the capture complex to move by capillary action through the membrane strip to the capture zone and to allow binding of the capture complex to the aptamer to form a complex;
e) further maintaining the membrane strip under conditions which allow movement of binding agents not immobilized in the capture zone into the absorbent zone; and
f) determining the amount of complex in the capture zone,

wherein the amount of analyte in the sample is directly related to the amount complex present in the capture zone.

**9.** The non-competitive assay method of claim 8, wherein the membrane strip comprises a material selected from the group consisting of cellulose, cellulose nitrate, cellulose acetate, glass fiber, nylon, polyelectrolyte, acrylic copolymer, polyethersulfone or the membrane strip comprises a single layer fusion matrix material;

**10.** The method of claim 8 or 9, wherein:

a) the amount of complex is determined as a ratio of the amount of first detectable label detected in the capture zone to the amount of a control detectable label detected in the capture zone; or
b) the aptamer is conjugated to a particle trapped within the membrane strip, and optionally wherein the control detectable label is in or on the particle.

**11.** The non-competitive assay method of any one of claims 8 to 10, wherein the aptamer is localized to a capture line within the capture zone, and optionally wherein the amount of binding agent detected in the capture line is normalized to the amount of binding agent that specifically binds a control analyte detected in a control capture line, and further optionally wherein the control analyte is added to the biological sample before the sample is administered to the application point of the membrane strip.

**12.** The non-competitive assay method of any one of claims 8 to 11, wherein the amount of analyte in the biological sample is determined by plotting the amount of binding agent detected against a response surface calculated from a plurality of analyte standards and adjusted using internal controls, and optionally wherein the response surface is adjusted using internal controls.

**13.** A kit for performing the non-competitive assay method of any one of claims 1 to 12 for an analyte, wherein the kit comprises:

a membrane strip comprising an application point, a capture zone, and an absorbent zone,
wherein the capture zone comprises an aptamer that selectively binds to a complex of the analyte and a binding agent, immobilized in or on the membrane strip and wherein the binding agent is not an aptamer.

**14.** The kit of claim 13:

a) wherein the membrane strip further comprises a conjugation zone, wherein the conjugation zone comprises the binding agent wherein the binding agent comprises an antibody immobilized in or on the membrane strip;
b) wherein the capture zone comprises immobilized control analyte; or
c) further comprising a sample collection apparatus, wherein the sample collection apparatus comprises binding agents that selectively bind the analyte.

**Patentansprüche**

**1.** Nicht-kompetitiver Assay zum quantitativen Messen der Menge eines Analyten in einer biologischen Probe aus einem Subjekt, Folgendes umfassend:

a) Umsetzen der biologischen Probe mit

(i) einem Bindungsmittel, das selektiv an den Analyten bindet, um einen Fangkomplex aus dem Bindungsmittel und dem Analyten zu bilden, wobei das Bindungsmittel kein Aptamer ist; und
(ii) ein Aptamer, das selektiv an den Analyten bindet, der an den Fangkomplex gebunden ist, um einen Komplex aus dem Bindungsmittel, dem Aptamer und dem Analyten zu bilden; und

b) Messen der Menge des Aptamer-gebundenen Komplexes;

wobei der Analyt ein Molekülmasse von weniger als 2.000 Dalton aufweist, und die Menge der Komplexbildung in direktem Zusammenhang mit der Menge des Analyten in der Probe steht.

**2.** Nicht-kompetitives Assayverfahren nach Anspruch 1, wobei

a) das Bindungsmittel einen Antikörper umfasst, und/oder
b) das Aptamer ein Nukleinsäureaptamer ist, oder ein Peptidaptamer ist, das selektiv an den Fangkomplex bindet.

**3.** Nicht-kompetitives Assayverfahren nach Anspruch 1 oder 2, wobei das Bindungsmittel mit einem ersten nachweisbaren Marker verknüpft ist und/oder das Aptamer mit einem zweiten nachweisbaren Marker verknüpft ist, und wahlweise:

a) wobei der erste nachweisbare Marker und der zweite nachweisbare Marker fluoreszierende Moleküle mit unterschiedlichen Exzitations- und Emissions-Wellenlängenkombinationen sind;
b) wobei der erste nachweisbare Marker und der zweite nachweisbare Marker ein Fluoreszenz-Resonanz-Energietransfer(FRET)-Donor-Akzeptor-Paar bilden; und/oder
c) wobei das Nukleinsäureaptamer-Bindungsmittel ein Paar aus Fluorophor und Quencher umfasst, wobei die Bildung des Komplexes dazu führt, dass das Quenchen oder Nichtquenchen des Flurophors nachweisbar ist.

**4.** Nicht-kompetitives Assayverfahren nach einem der vorhergehenden Ansprüche, wobei der Analyt ein Hormon, Arzneimittel, Arzneimittelmetabolit oder Schwermetallion ist.

**5.** Nicht-kompetitives Assayverfahren nach Anspruch 4, wobei das Arzneimittel ein Suchtmittel ist, zum Beispiel ein Opioid oder Opioidmetabolit, wie beispielsweise:

a) ein Opioid ausgewählt aus der Gruppe, bestehend aus Morphin, Kodein, Thebain, Heroin, Hydromorphon, Hydrocodon, Oxycodon, Oxymorphon, Desomorphin, Nicomorphin, Propoxyphen, Dipropanoylmorphin, Benzylmorphin, Ethylmorphin, Buprenorphin, Fentanyl, Pethidin, Meperidin, Methadon, Tramadol, Dextropropoxyphen und Kombinationen davon.
b) Oxycodon, Hydrocodon oder eine Kombination davon; oder

c) Noroxycodon, Oxymorphon, Hydromorphon, Norhydrocodon oder eine Kombination davon.

6. Nicht-kompetitives Assayverfahren nach Anspruch 5, wobei das Arzneimittel ein Suchtmittel ist und der Analyt THC oder ein Metabolit davon ist, oder wobei das Arzneimittel ein Opioid oder Opioidmetabolit ist und der Analyt Nikotin oder ein Metabolit davon ist.

7. Nicht-kompetitives Assayverfahren nach einem der Ansprüche 1 bis 6, umfassend einen Lateral-Flow-Immunassay.

8. Nicht-kompetitives Assayverfahren nach Anspruch 7, umfassend

a) wahlweise Zugeben von Bindungsmitteln zu der biologischen Probe;
b) Applizieren der biologischen Probe auf einen Membranstreifen, umfassend einen Applikationspunkt, eine wahlweise Konjugationszone, eine Fangzone und eine Absorptionszone, wobei die Konjugationszone das Bindungsmittel umfasst, wobei die Fangzone das Aptamer umfasst, welches immobilisiert in oder auf dem Membranstreifen vorliegt, wobei die biologische Probe an dem Applikationspunkt appliziert ist;
c) wahlweise Beibehalten des Membranstreifens unter Bedingungen, die es dem in der biologischen Probe vorliegenden Analyten ermöglichen, sich durch Kapillarwirkung durch den Membranstreifen bis zur Konjugationszone zu bewegen und die Bindung des Bindungsmittels an den Analyten zu ermöglichen, um einen Fangkomplex zu bilden;
d) Beibehalten des Membranstreifens unter Bedingungen, die es dem Fangkomplex ermöglichen, sich durch Kapillarwirkung durch den Membranstreifen bis zur Fangzone zu bewegen und die Bindung des Fangkomplexes an das Aptamer zu ermöglichen, um einen Komplex zu bilden;
e) des Weiteren Beibehalten des Membranstreifens unter Bedingungen, welche die Bewegung von Bindungsmitteln, die nicht in der Fangzone immobilisiert sind, in die Absorptionszone ermöglichen; und
f) Bestimmen der Menge des Komplexes in der Fangzone,

wobei die Menge an Analyt in der Probe in direktem Zusammenhang mit der Menge des Komplexes in der Fangzone steht.

9. Nicht-kompetitives Assayverfahren nach Anspruch 8, wobei der Membranstreifen ein Material umfasst, das aus der Gruppe ausgewählt ist, bestehend aus Zellulose, Zellulosenitrat, Zelluloseacetat, Glasfaser, Nylon, Polyelektrolyt, Acrylcopolymer, Polyethersulfon oder wobei der Membranstreifen ein aus einer Schicht bestehendes Fusionsmatrixmaterial umfasst.

10. Verfahren nach Anspruch 8 oder 9, wobei:

a) die Menge des Komplexes festgelegt ist als ein Verhältnis der Menge eines ersten nachweisbaren Markers, der in der Fangzone detektiert wird, zu der Menge eines nachweisbaren Kontrollmarkers, der in der Fangzone detektiert wird; oder
b) das Aptamer mit einem Partikel, welcher innerhalb des Membranstreifens eingeschlossen ist, konjugiert ist, und wahlweise wobei der nachweisbare Kontrollmarker in oder auf dem Partikel vorliegt.

11. Nicht-kompetitives Assayverfahren nach einem der Ansprüche 8 bis 10, wobei das Aptamer an einer Fanglinie innerhalb der Fangzone lokalisiert ist, und wahlweise wobei die Menge des Bindungsmittels, das in der Fanglinie detektiert wird, bis zu der Menge an Bindungsmittel, welches spezifisch an einen Kontrollanalyten bindet, der in einer Kontroll-Fanglinie detektiert wird, normalisiert wird, und des Weiteren wahlweise wobei der Kontrollanalyt zu der biologischen Probe zugesetzt wird, bevor die Probe an dem Applikationspunkt des Membranstreifens aufgetragen wird.

12. Nicht-kompetitives Assayverfahren nach einem der Ansprüche 8 bis 11, wobei die Menge des Analyten in der biologischen Probe bestimmt wird, indem die Menge des nachgewiesenen Bindungsmittels gegen eine Reaktionsfläche, die aus einer Vielzahl von Analytstandards berechnet wird, geplottet und unter Verwendung interner Kontrollen angepasst wird, und wahlweise wobei die Reaktionsfläche unter Verwendung interner Kontrollen angepasst wird.

13. Kit zum Durchführen des nicht-kompetitiven Assayverfahrens nach einem der Ansprüche 1 bis 12 für einen Analyten, wobei das Kit Folgendes umfasst:

einen Membranstreifen, umfassend einen Applikationspunkt, eine Fangzone und eine Absorptionszone, wobei die Fangzone ein Aptamer umfasst, das selektiv an einen Komplex aus dem Analyten und einem Bindungsmittel, welches in oder auf dem Membranstreifen immobilisiert vorliegt, bindet, und wobei das Bindungsmittel kein Aptamer ist.

**14.** Kit nach Anspruch 13:

a) wobei der Membranstreifen des Weiteren eine Konjugationszone umfasst, wobei die Konjugationszone das Bindungsmittel umfasst, wobei das Bindungsmittel einen Antikörper umfasst, der in oder an dem Membranstreifen immobilisiert ist;
b) wobei die Fangzone einen immobilisierten Kontrollanalyten umfasst; oder
c) des Weiteren umfassend eine Probensammelvorrichtung, wobei die Probensammelvorrichtung Bindungsmittel umfasst, die selektiv an den Analyten binden.

## Revendications

**1.** Dosage non compétitif pour mesurer quantitativement la quantité d'un analyte dans un échantillon biologique d'un patient, comprenant les étapes consistant à :

a) faire réagir l'échantillon biologique avec

(i) un agent de liaison qui se lie sélectivement à l'analyte pour former un complexe de capture de l'agent de liaison et de l'analyte, où l'agent de liaison n'est pas un aptamère, et
(ii) un aptamère qui se lie sélectivement à l'analyte lié au complexe de capture pour former un complexe de l'agent de liaison, de l'aptamère et de l'analyte, et

b) mesurer la quantité de complexe lié à l'aptamère ;

dans lequel l'analyte a un poids moléculaire inférieur à 2 000 daltons, et la quantité de formation du complexe est directement liée à la quantité de l'analyte dans l'échantillon.

**2.** Procédé de dosage non compétitif selon l'une quelconque de la revendication 1, dans lequel

a) l'agent de liaison comprend un anticorps ; et/ou
b) l'aptamère est un aptamère d'acide nucléique, ou un aptamère peptidique qui se lie sélectivement au complexe de capture.

**3.** Procédé de dosage non compétitif selon l'une quelconque de la revendication 1 ou 2, dans lequel l'agent de liaison est lié à un premier marqueur détectable et/ou l'aptamère est lié à un deuxième marqueur détectable, et facultativement :

a) dans lequel le premier marqueur détectable et le deuxième marqueur détectable sont des molécules fluorescentes ayant des combinaisons de longueurs d'onde d'excitation et d'émission distinctes ;
b) dans lequel le premier marqueur détectable et le deuxième marqueur détectable forment une paire donneur/accepteur de transfert d'énergie de résonance par fluorescence (TERF) ; et/ou
c) dans lequel l'agent de liaison à l'aptamère d'acide nucléique comprend une paire fluorophore et désactivateur, où la formation du complexe entraîne la désactivation ou la non-désactivation détectable du fluorophore.

**4.** Procédé de dosage non compétitif selon l'une quelconque des revendications précédentes, dans lequel l'analyte est une hormone, un médicament, un métabolite de médicament ou un ion de métal lourd.

**5.** Procédé de dosage non compétitif selon la revendication 4, dans lequel le médicament est un médicament prescrit sur ordonnance, par exemple un opioïde ou un métabolite d'opioïde, notamment :

a) un opioïde choisi dans le groupe constitué par la morphine, la codéine, la thébaïne, l'héroïne, l'hydromorphone, l'hydrocodone, l'oxycodone, l'oxymorphone, la désomorphine, la nicomorphine, le propoxyphène, la dipropanoylmorphine, la benzylmorphine, l'éthylmorphine, la buprénorphine, le fentanyl, la péthidine, la mépéridine, la

méthadone, le tramadol, le dextropropoxyphène et les combinaisons de ceux-ci,
b) l'oxycodone, l'hydrocodone ou une combinaison de celles-ci ; ou
c) la noroxycodone, l'oxymorphone, l'hydromorphone, la norhydrocodone ou une combinaison de celles-ci.

**6.** Procédé de dosage non compétitif selon la revendication 5, dans lequel le médicament est un médicament prescrit sur ordonnance et l'analyte est le THC ou un métabolite de celui-ci, ou dans lequel le médicament est un opioïde ou un métabolite d'opioïde et l'analyte est la nicotine ou un métabolite de celle-ci.

**7.** Procédé de dosage non compétitif selon l'une quelconque des revendications 1 à 6, comprenant un immunodosage à flux latéral.

**8.** Procédé de dosage non compétitif selon la revendication 7, comprenant les étapes consistant à :

a) facultativement ajouter des agents de liaison à l'échantillon biologique ;
b) appliquer l'échantillon biologique sur une bande de membrane comprenant un point d'application, une zone de conjugaison facultative, une zone de capture et une zone d'absorption, où la zone de conjugaison comprend l'agent de liaison, où la zone de capture comprend l'aptamère immobilisé dans ou sur la bande de membrane, où l'échantillon biologique est appliqué sur le point d'application ;
c) facultativement maintenir la bande de membrane dans des conditions qui permettent à l'analyte présent dans l'échantillon biologique de traverser, par capillarité, la bande de membrane jusqu'à la zone de conjugaison, et de permettre la liaison de l'agent de liaison à l'analyte pour former un complexe de capture ;
d) maintenir la bande de membrane dans des conditions qui permettent au complexe de capture de traverser, par capillarité, la bande de membrane jusqu'à la zone de capture, et de permettre la liaison du complexe de capture à l'aptamère pour former un complexe ;
e) maintenir également la bande de membrane dans des conditions qui permettent le déplacement des agents de liaison non immobilisés dans la zone de capture dans la zone d'absorption ; et
f) déterminer la quantité de complexe dans la zone de capture,

dans lequel la quantité d'analyte dans l'échantillon est directement liée à la quantité de complexe présente dans la zone de capture.

**9.** Procédé de dosage non compétitif selon la revendication 8, dans lequel la bande de membrane comprend un matériau choisi dans le groupe constitué par la cellulose, le nitrate de cellulose, l'acétate de cellulose, la fibre de verre, le nylon, le polyélectrolyte, le copolymère acrylique, le polyéthersulfone ou la bande de membrane comprend un matériau matriciel de fusion monocouche.

**10.** Procédé selon la revendication 8 ou 9, dans lequel :

a) la quantité de complexe est déterminée sous la forme d'un rapport de la quantité de premier marqueur détectable détecté dans la zone de capture à la quantité d'un marqueur de contrôle détectable détecté dans la zone de capture ; ou
b) l'aptamère est conjugué à une particule piégée dans la bande de membrane, et facultativement dans lequel le marqueur de contrôle détectable est dans ou sur la particule.

**11.** Procédé de dosage non compétitif selon l'une quelconque des revendications 8 à 10, dans lequel l'aptamère est localisé sur une ligne de capture à l'intérieur de la zone de capture, et facultativement dans lequel la quantité d'agent de liaison détecté sur la ligne de capture est normalisée en fonction de la quantité d'agent de liaison qui se lie spécifiquement à un analyte de contrôlé détecté sur une ligne de capture de contrôle, et également facultativement dans lequel l'analyte de contrôle est ajouté à l'échantillon biologique avant d'administrer l'échantillon sur le point d'application de la bande de membrane.

**12.** Procédé de dosage non compétitif selon l'une quelconque des revendications 8 à 11, dans lequel la quantité d'analyte dans l'échantillon biologique est déterminée en représentant graphiquement la quantité d'agent de liaison détecté en fonction d'une surface de réponse calculée d'après une pluralité d'étalons analytiques et ajustée à l'aide de contrôles internes, et facultativement dans lequel la surface de réponse est ajustée à l'aide de contrôles internes.

**13.** Kit pour effectuer le procédé de dosage non compétitif selon l'une quelconque des revendications 1 à 12 pour un analyte, lequel kit comprend :

une bande de membrane comprenant un point d'application, une zone de capture et une zone d'absorption, dans lequel la zone de capture comprend un aptamère qui se lie sélectivement à un complexe de l'analyte et d'un agent de liaison, immobilisé dans ou sur la bande de membrane et dans lequel l'agent de liaison n'est pas un aptamère.

**14.** Kit selon la revendication 13 :

a) dans lequel la bande de membrane comprend en outre une zone de conjugaison, où la zone de conjugaison comprend l'agent de liaison, où l'agent de liaison comprend un anticorps immobilisé dans ou sur la bande de membrane ;
b) dans lequel la zone de capture comprend un analyte de contrôle immobilisé ; ou
c) comprenant en outre un appareil de collecte des échantillons, où l'appareil de collecte des échantillons comprend des agents de liaison qui se lient sélectivement à l'analyte.

**Figures 1A-1B**

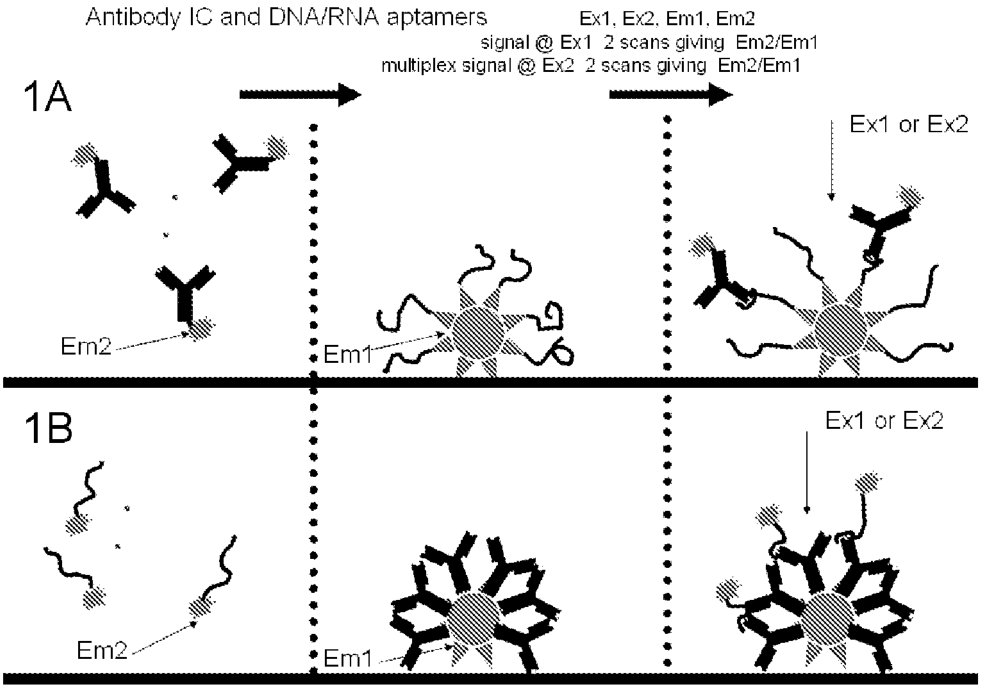

**Figures 2A-2B**

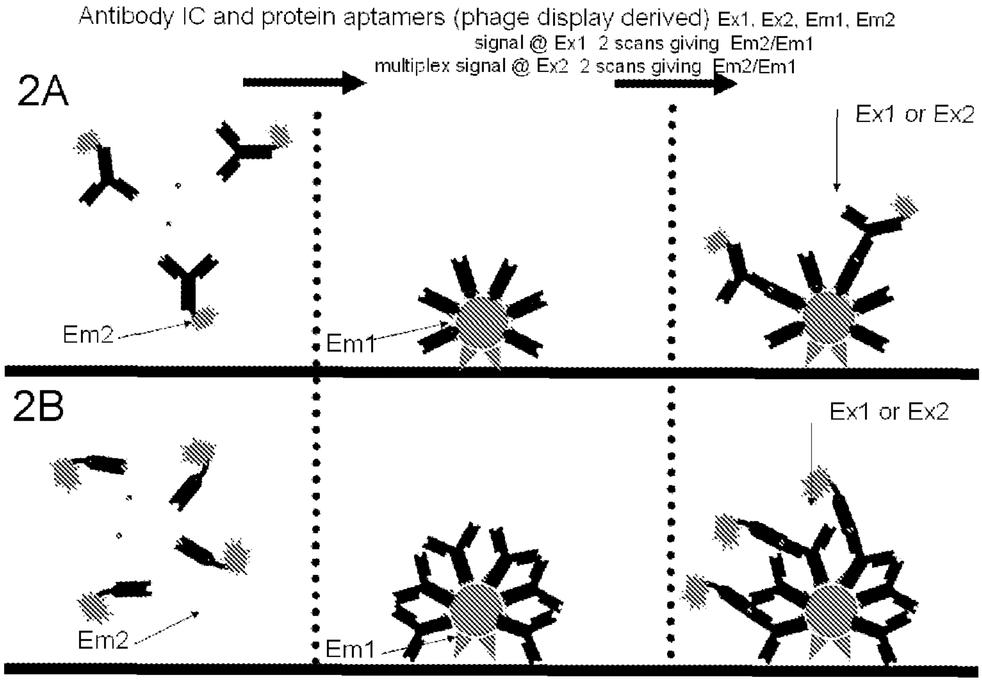

Figures 3A-3B

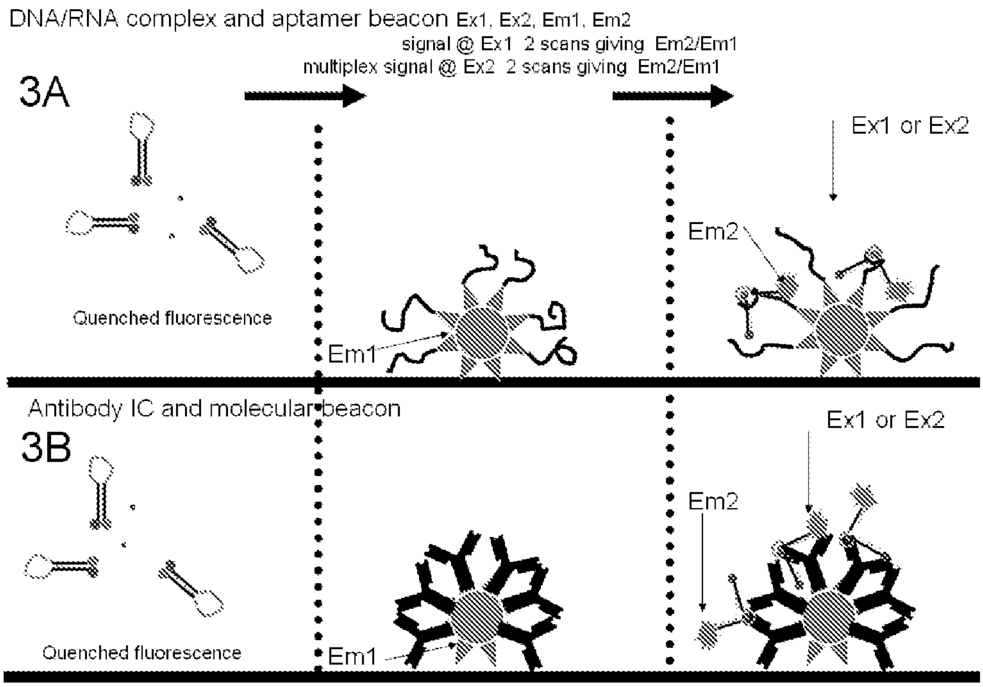


Figures 3C

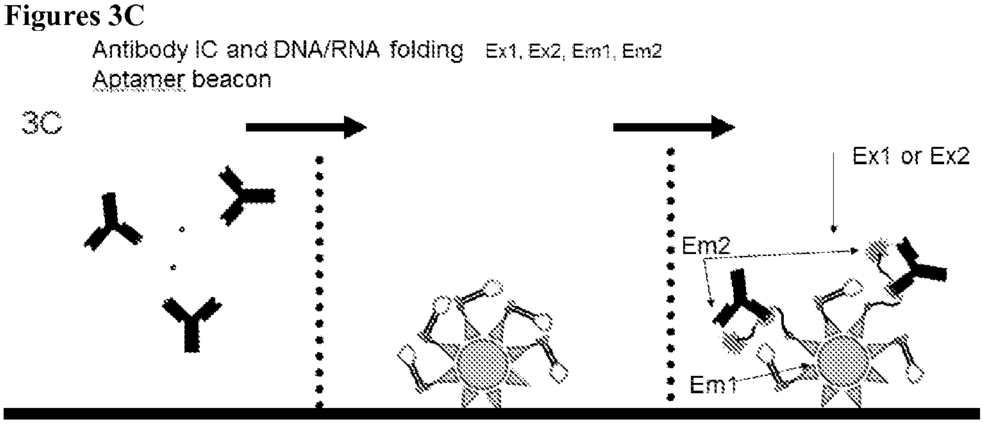

**Figure 4**

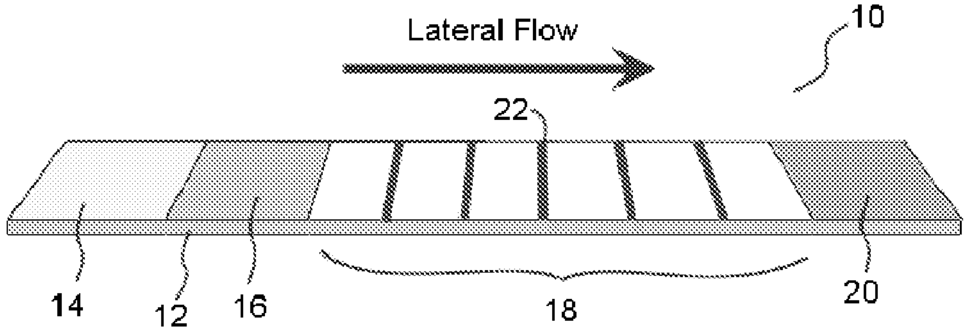

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5476766 A
- US 5503978 A
- US 5631146 A
- US 5731424 A
- US 5780228 A
- US 5792613 A
- US 5795721 A
- US 5846713 A
- US 5858660 A
- US 5861254 A
- US 5864026 A
- US 5869641 A
- US 5958691 A
- US 6001988 A
- US 6011020 A
- US 6013443 A
- US 6020130 A
- US 6028186 A
- US 6030776 A
- US 6051698 A
- US 5043265 A


### Non-patent literature cited in the description


- **CHRISTO et al.** *Pain Physician,* 2011, vol. 14, 123-143
- **JAYASENA.** *Clin. Chem.,* 1999, vol. 45, 1628-50
- **MASCINI et al.** *Angew. Chem. Int. Ed.,* 2012, vol. 51, 1316-1332
- **WYLIE et al.** *Proc. Natl. Acad. Sci. USA,* 1992, vol. 89, 4104-4108
- **BARBAS et al.** *Proc. Natl. Acad. Sci. USA,* 1993, vol. 90, 6385-6389
- **BLAKE DA et al.** *Biosensors & Bioelectronics,* 2001, vol. 16, 799-809
- **CHO et al.** *Annu Rev Anal Chem,* 2009, vol. 2, 241-64
- **HAMAGUCHI N et al.** *Anal Biochem.,* 2001, vol. 294 (2), 126-31
- **LI JJ et al.** *Biochem Biophys Res Commun.,* 2002, vol. 292 (1), 31-40
- **MOELLER et al.** *Mayo Clin. Proc.,* 2008, vol. 83 (1), 66-76
- **MONTGOMERY ; DOUGLAS C.** Design and Analysis of Experiments: Response surface method and designs. John Wiley and Sons, Inc, 2005
- **MAYER.** *Anew. Chem. Int. Ed.,* 2009, vol. 48, 2672-2689
- **STOLTENBURG et al.** *Anal. Bioanal. Chem.,* 2005, vol. 383, 83-91